# EUROPÄISCHE PATENTSCHRIFT

(11) **EP 2 371 806 B1**
(45) Veröffentlichungstag und Bekanntmachung des Hinweises auf die Patenterteilung: **12.07.2017**
(21) Anmeldenummer: 11159825.6
(22) Anmeldetag: 25.03.2011
(51) Int. Cl.: C07C 68/02, C07C 69/96, C08G 64/30

(54) **Verfahren zur Herstellung von Diarylcarbonaten und Polycarbonaten**
Method for manufacturing diaryl carbonates and polycarbonates
Procédé de fabrication de diarylcarbonates et de polycarbonates

(30) Priorität: 30.03.2010 EP 10158364
(43) Veröffentlichungstag der Anmeldung: 05.10.2011
(73) Patentinhaber: Covestro Deutschland AG, 51373 Leverkusen (DE)
(72) Erfinder: Ooms, Pieter, 47800 Krefeld (DE); Bulan, Andreas, 40764 Langenfeld (DE)
(74) Vertreter: Levpat

(56) Entgegenhaltungen:
- WO-A2-03/014419
- DE-A1-102007 058 701

## Beschreibung

Die vorliegende Erfindung betrifft ein Verfahren zur kontinuierlichen Herstellung von Diarylcarbonaten aus Phosgen und wenigstens einer Monohydroxyverbindung (Monophenol) in Gegenwart von Katalysatoren, sowie deren Verwendung zur Herstellung von Polycarbonaten. Der dabei anfallende Chlorwasserstoff wird durch elektrochemische Oxidation zu Chlor umgesetzt, wobei das Chlor zur Herstellung des Phosgens rückgeführt wird. Insbesondere besteht das Verfahren in der Verwertung des anfallenden Chlorwasserstoffs für das Diphenylcarbonat-Herstellungsverfahren (DPC-Verfahren).

Es ist bekannt, dass Diarylcarbonate, insbesondere Diphenylcarbonat durch Phasengrenzflächenphosgenierung (Schotten-Baumann-Reaktion) von Monophenolen in einem inerten Lösungsmittel in Gegenwart von Alkali und einem Katalysator hergestellt werden können. Dabei wirkt sich die Verwendung von Lösungsmitteln und Natronlauge nachteilig aus, da durch die wässrige Lauge eine teilweise Verseifüng von Phosgen oder Chlorkohlensäureester stattfinden kann, große Mengen Kochsalz als Nebenprodukt anfallen und das Lösungsmittel und Katalysator zurückgewonnen werden müssen.

Aus dem Grund ist auch die Herstellung von Diarylcarbonaten und insbesondere Diphenylcarbonat durch Reaktion von Monophenolen und Phosgen ohne Alkali und ohne Verwendung von Lösungsmitteln in Gegenwart von einem Katalysator durch den Direktphosgenierungsprozess untersucht worden und prinzipiell in der Literatur beschrieben.

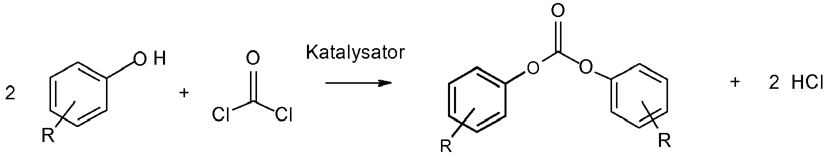

Vorschläge für Verfahren ohne Lösungsmittel mit löslichen Katalysatoren werden in US 2 837 555, 3 234 263 und 2 362 865 beschrieben.

Auch zu der Verwendung von heterogenen, nicht löslichen Katalysatoren, die eine Aufarbeitung des Reaktionsgemisches wesentlich erleichtern, wurden Vorschläge gemacht. So wird in der EP 516 355 A2 vor allem Aluminiumtrifluorid empfohlen, das auf Träger wie Alumosilikate aufgebracht wird. Die Synthese von Aluminiumfluorid ist jedoch durch die Handhabung von Fluor oder Flußsäure sehr aufwendig und teurer.

Weiter werden in der WO 91/06526 Metallsalze auf porösen Trägern als Katalysatoren für die erfindungsgemäßen Umsetzungen beschrieben. Eine vollkontinuierliche Phosgenierung von Phenol an solchen Katalysatoren ist nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht. Deshalb wurden nicht-geträgerte Katalysatoren vorgeschlagen, die den großen Vorteil haben, dass man den Katalysator sehr leicht abtrennen kann und keine Verunreinigungen im rohen Reaktionsprodukt verbleiben. Die Aufarbeitung wird dadurch wesentlich vereinfacht.

So wurden Verfahren zur Herstellung von Diarylcarbonaten durch Phosgenierung von Monophenolen in Gegenwart von heterogenen Katalysatoren wie Aktivkohlen (EP 483 632), Aluminiumoxiden (EP 635 477), Alumosilikaten (EP 635 476), Metalloxiden (EP 645 364), Metallaten (EP 691 326), Hartstoffe (EP 722 930) und Mischhydroxiden (DE 10 2008 050 828) als auch in Gegenwart von homogenen Katalysatoren wie Metallsalzen (US 634622), aromatischen Stickstoffheterocyclen (D-OS 2 447 348) und Organophosphorverbindungen (US-5136077) sowohl in der Flüssigphase (EP 757 029, EP 791 574) als auch in der Gasphase (EP 808 821) beschrieben. DE-A-10 2007 058 701 offenbart ein Verfahren zur Herstellung von Diarylcarbonaten und Verarbeitung mindestens eines Teils der dabei anfallenden Alkalichloridhaltigen Lösung in einer nachgeschalteten Alkalichlorid-Elektrolyse. Nach der Synthese des Diarylcarbonats wird das Diarylcarbonat als Mischung mit dem eingesetzten Monophenol und gegebenenfalls Katalysator oder gegebenenfalls in Form seiner Lösung in dem bei der Synthese verwendeten organischen Lösungsmittel, beispielsweise Chorbenzol, abgetrennt.

Zum Erhalt des hochreinen Diarylcarbonats kann eine Reinigung durch Destillation und / oder Kristallisation erfolgen. Beispielsweise erfolgt dies durch eine oder mehrere hintereinander geschaltete Destillationskolonnen bei der gegebenenfalls das Lösungsmittel vom Diarylcarbonat abgetrennt wird.

Diese Reinigungsstufe bzw. Stufen können beispielsweise kontinuierlich so geführt werden, dass die Sumpftemperatur bei der Destillation 150°C bis 310°C, bevorzugt 160 bis 230°C beträgt. Der zur Durchführung dieser Destillation angewendete Druck beträgt dabei insbesondere 1 bis 1000 mbar, bevorzugt 5 bis 100 mbar.

Die so gereinigten Diarylcarbonate zeichnen sich durch besonders hohe Reinheit (GC >99,95%) und sehr gutes Umesterungsverhalten aus, so dass hieraus anschließend ein Polycarbonat in exzellenter Qualität hergestellt werden kann.

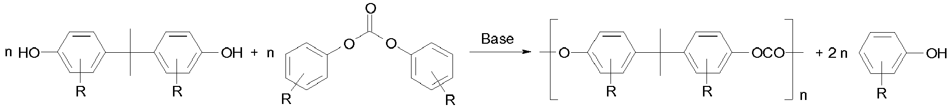

Die Verwendung der Diarylcarbonate zur Herstellung von aromatischen Oligo- / Polycarbonaten nach dem Schmelzumesterungsverfahren ist literaturbekannt und beispielsweise in der Encyclopedia of Polymer Science, Vol. 10 (1969), Chemistry and Physics of Polycarbonates, Polymer Reviews, H. Schnell, Vol. 9, John Wiley and Sons, Inc. (1964), S. 50/51 oder der US-A-5 340 905 beschrieben.

Die Nutzung des bei der Herstellung von Diphenylcarbonat durch Direktphosgenierung von Phenol gebildeten Chlorwasserstoffs kann z.B. durch Vermarktung der wässrigen Lösung (Salzsäure) oder durch Verwendung bei Synthesen anderer chemischer Produkte erfolgen. Die anfallenden Mengen Chlorwasserstoff können jedoch nicht immer vollständig vermarktet oder für andere Synthesen eingesetzt werden. Außerdem kann Chlorwasserstoff für Synthesen nur dann eingesetzt werden, wenn er zuvor entsprechend gereinigt wird. Andererseits ist die Vermarktung meist nur dann wirtschaftlich, wenn der Chlorwasserstoff bzw. die Salzsäure nicht über weite Wege transportiert werden müssen.

Eine der gängigsten Nutzungsmöglichkeiten für den anfallenden Chlorwasserstoff ist daher die Verwendung als Rohstoff bei der PVC-Herstellung, bei dem die Oxychlorierung von Ethylen mit Chlorwasserstoff zu Ethylendichlorid erfolgt. Dennoch ist diese Verfahrensweise nicht generell anwendbar, da die entsprechenden Herstellbetriebe sich meistens nicht in unmittelbarer Umgebung einer Diarylcarbonatherstellung befinden. Die Entsorgung des Chlorwasserstoffes, z.B. durch Neutralisation mit Lauge, ist aus wirtschaftlicher und ökologischer Sicht unattraktiv.

Ein Recyclingverfahren für den Chlorwasserstoff und die Rückführung des Chlors in den Diphenylcarbonat-Produktionsprozess, in dem der Chlorwasserstoff anfällt, ist daher die angestrebte Verfahrensweise.

Eine Übersicht über elektrochemische Recycling-Verfahren wird in dem Artikel "Chlorine Regeneration from Anhydrous Hydrogen Chloride" von Dennie Turin Mah, veröffentlicht in "12^{th} International Forum Electrolysis in Chemical Industry - Clean and Efficient Processing Electrochemical Technoogy for Synthesis, Separation, Recycle and Environmental Improvement, October 11-15, 1998, Sheraton Sand Key, Clearwater Beach, FL", gegeben.

Die Rückführung von Chlorwasserstoff durch elektrochemische Oxidation unter Bildung von Chlor und Wasserstoff ist in LU 88 569 und EP 1 112 997 beschrieben. Nachteilig ist die geringe Stromausbeute und die Produktion von Wasserstoff, der für das Polycarbonatherstellungsprozess keine Verwendung hat.

Ausgehend vom oben geschilderten Stand der Technik bestand die Aufgabe der vorliegenden Erfindung darin, ein Diarylcarbonat-Herstellungsverfahren bereitzustellen, welches Produkte in hoher Reinheit und guter Ausbeute liefert und eine Reduzierung der Umweltbelastung bzw. Abwasserproblematik in den Klärwerken durch maximierte Rückführung von Nebenprodukten, die aus der Polycarbonat-Produktion stammen, erreicht wird. Insbesondere sollte bei dem Recycling die Umsetzung von Chlorwasserstoff zu Chlor mit minimalem Energieeinsatz und daher resourcenschonend erfolgen.

Es wurde nun gefunden, dass sich Chlorwasserstoff besonders günstig wiederverwerten lässt, wenn dieser Chlorwasserstoff durch elektrochemische Oxidation über die sogenannte Sauerstoffverzehrkathode wieder zu Chlor umgesetzt wird und zur Herstellung von Phosgen genutzt wird.

Der bei der kontinuierlichen Herstellung von Diarylcarbonaten durch Reaktion von Monophenolen und Phosgen in Gegenwart von Katalysatoren anfallende Chlorwasserstoff kann ohne aufwendige Reinigung als wässrige Lösung gegebenenfalls nach einfacher Behandlung mit Aktivkohle direkt einer elektrochemischen Oxidation zu Chlor und Wasser zugeführt werden, wobei das Chlor zur Herstellung des Phosgens rückgeführt werden kann.

Gegenstand der vorliegenden Erfindung ist demnach ein Verfahren zur Herstellung von Diarylcarbonaten ausgehend von Monophenolen und Carbonyldihalogenid, das dadurch gekennzeichnet ist, dass der dabei entstehende Halogenwasserstoff durch Elektrolyse mit einer Gasdiffusionselektrode als Kathode in das Halogen und dieses wiederum nach Isolierung und Reaktion mit Kohlenmonoxid zum Carbonyldihalogenid umgesetzt wird, welches anschließend wieder mit dem Monophenol zur Herstellung des Diarylcarbonats eingesetzt wird. Das Monophenol, das bei der Herstellung von lösungsmittelfreiem Polycarbonat durch Umesterung von Diarylcarbonaten und Bisphenolen freigesetzt wird, kann wiederum zur Herstellung des Diarylcarbonats verwendet werden.

Weiterhin betrifft die Anmeldung ein Verfahren in dem das hergestellte Diarylcarbonat zur Herstellung von Polycarbonat verwendet wird.

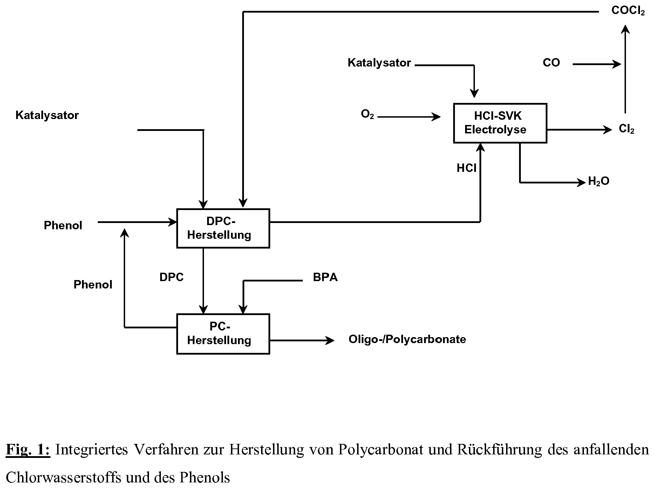

Das erfindungsgemäße Gesamtverfahren ist flexibel, einfach in der Durchführung und liefert Produkte in hoher Reinheit, die für den Gesamtprozess außerordentlich wichtig sind unter gleichzeitiger Reduzierung der Umweltbelastung durch Wiederverwendung, umfassend folgende Prozessschritte (vgl. Fig. 1):
(a) Herstellung von Phosgen durch Umsetzung von Chlor mit Kohlenmonoxid,
(b) Umsetzung des gemäß Schritt (a) gebildeten Phosgens mit wenigstens einem Monophenol in Gegenwart eines Katalysators, und gegebenenfalls organischem Lösungsmittel unter Bildung wenigstens eines Diarylcarbonats und von Chlorwasserstoff,
(c) Abtrennung und Aufarbeitung des in Schritt (b) gebildeten Diarylcarbonats
(d) Abtrennung und gegebenenfalls Reinigung des gemäß Schritt (b) gebildeten Chlorwasserstoffs,
(e) Herstellung einer wässrigen Lösung des Chlorwasserstoffs (Salzsäure)
(f) gegebenenfalls Reinigung der wässrigen Lösung des Chlorwasserstoffs,
(g) elektrochemische Oxidation wenigstens eines Teils der wässrigen Chlorwasserstoff-Lösung aus (e) oder (f) zu Chlor unter Bildung von Wasser
(h) Rückführung wenigstens eines Teils des gemäß Schritt (g) hergestellten Chlors in die Herstellung von Phosgen gemäß Schritt (a),
wobei die elektrochemische Oxidation in Schritt (g) unter Verwendung einer Gasdiffusionselektrode erfolgt.

Die Durchführung der Elektrolyse mit einer Gasdiffusionselektrode als Sauerstoffverzehtkathode führt zu einer erheblichen Reduzierung des Energieverbrauchs im Vergleich mit einer klassischen Elektrolyse, die zudem neben Chlor noch Wasserstoff produziert, der in eine Diaryl- oder Polycarbonatherstellung keine Verwendung hat.

In einer besonders bevorzugten Ausführungsform wird wenigstens ein Teil des gemäß Schritt (c) hergestellten Diarylcarbonats mit einem Bisphenol zum Oligo-/Polycarbonat und dem Monophenol umgesetzt (die sogenannte Umesterungsreaktion). Das bei der Umesterung entstehende Monophenol kann in einer weiteren bevorzugten Ausführungsform wiederum in Schritt (b) eingesetzt werden.

Das erfindungsgemäße Verfahren ist ein Verfahren zur Herstellung von Diarylcarbonaten und der Elektrolyse einer wässrigen Lösung aus Chlorwasserstoff zur Rückgewinnung von Chlor für die Synthese von Phosgen als Ausgangsprodukt für die Diarylcarbonatherstellung.

Im ersten Schritt (a) des erfindungsgemäßen Verfahrens erfolgt die Herstellung von Phosgen durch Umsetzung von Chlor mit Kohlenmonoxid. Die Synthese von Phosgen ist hinlänglich bekannt und ist z.B. in Ullmanns Enzyklopädie der industriellen Chemie, 3. Auflage, Band 13, Seite 494-500 dargestellt. Im technischen Maßstab wird Phosgen überwiegend durch Umsetzung von Kohlenmonoxid mit Chlor bevorzugt an Aktivkohle als Katalysator hergestellt. Die stark exotherme Gasphasenreaktion erfolgt bei Temperaturen von mindestens 250°C bis maximal 600°C in der Regel in Rohrbündelreaktoren. Die Abführung der Reaktionswärme kann auf unterschiedliche Weise erfolgen, beispielsweise durch ein flüssiges Wärmetauschmittel, wie z.B. in WO 03/072237 beschrieben, oder durch Siedekühlung über einen Sekundärkühlkreislauf unter gleichzeitiger Nutzung der Reaktionswärme zur Dampferzeugung, wie z.B. in US 4 764 308 offenbart.

Aus dem gemäß Schritt (a) gebildeten Phosgen wird durch Umsetzung mit wenigstens einem Monophenol in einem nächsten Verfahrensschritt (b) wenigstens ein Diarylcarbonat gebildet. Der Verfahrensschritt (b) wird nachfolgend auch als Phosgenierung bezeichnet. Die Umsetzung erfolgt unter Bildung von Chlorwasserstoff als Nebenprodukt.

Die Synthese von Diarylcarbonaten ist ebenfalls aus dem Stand der Technik hinlänglich bekannt, wobei in der Regel das Monophenol in einem stöchiometrischen Überschuss, bezogen auf das Phosgen, eingesetzt wird. Üblicherweise findet die Phosgenierung gemäß (b) in der Flüssigphase statt, wobei das Phosgen und das Monophenol in der Schmelze, gegebenenfalls in einem Lösemittel gelöst sein können. Bevorzugte Lösungsmittel sind chlorierte aromatische Kohlenwasserstoffe, wie beispielsweise Chlorbenzol, o-Dichlorbenzol, p-Dichlorbenzol, Trichlorbenzole, die entsprechenden Chlortoluole oder Chlorxylole, Toluol, Xylole und das Monophenol selbst.

Besonders bevorzugt ist das geschmolzene Monophenol als Lösungsmittel.

In einer anderen, bevorzugten Ausführungsform findet die Phosgenierung in der Gasphase statt. Die Gasphasenphosgenierung ist z.B. in US 5 831 111 beschrieben.

Für das erfindungsgernäße Verfahren geeignete Monophenole sind Phenole der Formel (I) 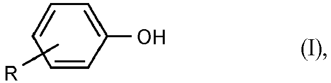 worin
- R: Wasserstoff, Halogen oder ein verzweigter oder unverzweigter C₁- bis C₉-Alkylrest, C₁- bis C₉-Alkoxyrest oder C₁ bis C₉-Alkoxycarbonylrest ist.

Bevorzugt sind also Phenol, Alkylphenole wie Kresole, p-tert.-Butylphenol, p-Cumylphenol, p-n-Octylphenol, p-iso-Octylphenol, p-n-Nonylphenol und p-iso-Nonylphenol, Halogenphenole wie p-Chlorphenol, 2,4-Dichlorphenol, p-Bromphenol, 2,4,6-Tribromphenol, Anisol und Salicylsäuremethyl oder -phenylester. Besonders bevorzugt ist Phenol.

Für das erfindungsgemäße Verfahren können heterogene als auch homogene Katalysatoren eingesetzt werden.

Als heterogene Katalysatoren können Aktivkohlen (EP 483 632), Aluminiumoxide (EP 635 477), Alumosilikate (EP 635 476), Metalloxide (EP 645 364), Metallate (EP 691 326) Hartstoffe (EP 722 930) und Mischhydroxide (DE 10 2008 050 828) sowohl in der Flüssigphase (EP 757 029, EP 791 574) als auch in der Gasphase (EP 808 821) eingesetzt werden.

Als homogene Katalysatoren können Metallsalze, deren Alkylate, aromatische Stickstoffheterocyclen oder Organophosphorverbindungen eingesetzt werden.

Es können ein oder mehrere, aktivierte oder nicht-aktivierte Katalysatoren eingesetzt werden.

Geignete Katalysatoren für Schritt b) des erfindungsgemäßen Verfahren sind:
a) Aktivkohlen mit nach der BET-Methode bestimmten Oberflächen von 200 bis 3000 m2/g, die aus Sägemehl und anderen Holzabfällen, Stroh, Kohlensorten, Nußschalen, Mineralölteeren, Lignin, Polysacchariden, Polyacrylnitril, Knochen, Torf oder Koksprodukte aus Braun oder Steinkohlen, bevorzugt aus Holz, Cellulose, Lignin, Bitumen- oder Braunkohle, Torf oder Steinkohlenkoks hergestellt wurde.
b) Alumosilikaten, ausgewählt aus der Gruppe der Zeolithe der allgemeinen Formel

   M_{2/n} O. x SiO₂. Al₂O₃. y H₂O

   in welcher
   M für Kationen wie Protonen oder Metallkationen des Periodensystems der Elemente nach Mendelejew steht,
   n für die Wertigkeit des Kations steht,
   x für das Mol-Verhältnis SiO₂.Al₂O₃ steht, wobei
   x eine Zahl von 1,0-50,0 sein kann, und
   y für eine Zahl von 0 - 9 steht,
   oder zeolith-ähnliche Verbindungen wie ALPO's und SAPO's oder Schichtsilikate des Kaolin-, Serpentin-, Montmorillonit- und Bentonit-Typs oder "pillared clays" oder SiO₂/Al₂O₃-Fällungskatalysatoren,
c) Aluminiumoxide oder y-Aluminiumoxide mit nach der BET-Methode bestimmten Oberflächen von 2 bis 500 m²/g,
d) Oxid(e) von Metallen der Gruppe IVB des periodischen Systems wie beispielsweise Oxide des Titans, Zirkoniums oder Hafniums mit nach der BET-Methode bestimmten Oberflächen von 2 bis 500 m²/g,
e) Metallate der allgemeinen Formel

   AₓB_{y}O_{z}

   in welcher
   A für ein mono-, di und/oder trivalentes Metallkation steht und
   B für ein tri-, tetra-, penta- undloder hexavalentes Metallkation steht und
   x für eine Zahl von 1 bis 4 steht und
   y für eine Zahl von 1 oder 2 steht und
   z für eine Zahl von 3, 6, 7 oder 9 steht,
f) Hartstoffen mit metallartigen Eigenschaften (Keramikvorprodukte) der allgemeinen Formel

   AₓB_{y}C_{z}D_{w}

   in welcher
   A für ein Element aus den Gruppen 3 bis 10, 13 und 14 des periodischen Systems der Elemente (IUPAC Notation) steht und
   B für ein Element aus den Gruppen 13, 14, 15 und 16 mit Ausnahme von Sauerstoff steht,
   C für ein Element aus den Gruppen 14 und 15 steht und
   D für ein Element aus den Gruppen 14 und 15 steht und
   x für eine Zahl von 1 bis 4 steht und
   y für eine Zahl von 1 bis 4 steht und
   z für eine Zahl von 0 bis 4 steht und
   w für eine Zahl von 0 bis 4 steht
   wobei A, B, C und D jeweils aus verschiedenen Gruppen oder bei gleicher Gruppe aus verschiedenen Perioden stammen, mit der Maßgabe, dass A verschieden von Aluminium ist, wenn B Kohlenstoff ist und gleichzeitig z und w gleich 0 sind,
g) Mischhydroxide der allgemeinen Formel (III)

   [M(II)₁₋ₓ M(III)ₓ M(IV)_{y} (OH)₂]Aⁿ⁻_{z/n}·mH₂O (III),

   in welcher
   M (II) für ein divalentes Metallkation steht und
   M (III) für ein trivalentes Metallkation steht und
   M(IV) für ein tetravalentes Metallkation steht und
   x für eine Zahl von 0,1 bis 0,5 steht und
   y für eine Zahl von 0 bis 0,5 steht
   z für 1 + y steht und
   m für eine Zahl von 0 bis 1 steht
   A für ein anorganisches Anion wie OH⁻, NO₃⁻, CO₃²⁻, SO₄²⁻, CrO₄²⁻ oder Cl⁻ steht.
   n für 1 bis 2 steht
h) aromatische heterocyclische Stickstoffbasen und deren Salze, wie z.B. Pyridin, Imidazol und Pyridin.HCl-Salz
i) Metallsalze, -halogenide, -alkylate und -phenolate wie z.B. AlCl₃, AlF₃, ZrCl₄, TiCl₄, VCl₃, VCl₄ oder Ti(OC₆H₅)₄,
j) Organophosphorverbindungen, homogen oder gegebenenfalls polymergebunden

Gegebenenfalls können die homogenen Katalysatoren auf einem inerten Träger aufgebracht werden, wie in JP 695219, WO 91/06526, US 5 424 473 und EP 516 355 beschrieben.

Bevorzugt sind Aluminiumoxid, Titanoxid, Zirkonoxid, Calciumtitanat, Magnesiumtitanat und Magnesiumaluminiumhydrotalcit, die im neuen Verfahren vorzugsweise als Festbett eingesetzt werden, und Titanchlorid, Zirkonchlorid, Aluminiumchlorid und Titanphenolat, die als homogene Katalysatoren eingesetzt werden.

Besonders bevorzugt sind Aluminiumoxid und TiCl₄.

Der Katalysator wird in Mengen von 0,5 bis 100 Gew.-% bezogen auf die Menge an Monohydroxyverbindung (Monophenol), bei nicht vollkontinuierlicher Fahrweise bzw. mit Belastungen von 0,1 bis 20 g Monohydroxyverbindung (Monophenol) pro g Katalysator pro Stunde bei vollkontinuierlicher Fahrweise, einge-setzt.

Phosgen kann in dem Verfahrensschritt b) flüssig, gasförmig oder gegebenenfalls gelöst in einem inerten Lösungsmittel eingesetzt werden.

In dem neuen Verfahren in Schritt b) gegebenenfalls verwendbare inerte organische Lösungsmittel sind beispielsweise Toluol, Chlorbenzol, Dichlorbenzol und Chlortoluol, bevorzugt ist Phenol selbst.

Wird ein Phosgenierung in der Gasphase gewählt, so erfolgt die Umsetzung oberhalb der Siedetemperatur des Phenols, bevorzugt innerhalb einer mittleren Kontaktzeit von 0,5 bis 5 Sekunden und bei Temperaturen von 180 bis 500°C.

Bei der Phosgenierung in der Flüssigphase werden üblicherweise Temperaturen von 20 bis 240°C und Drücke von 1 bis ca 50 bar eingesetzt. Die Phosgenierung in der Flüssigphase kann einstufig oder mehrstufig durchgeführt werden, wobei im allgemeinem Phenol im stöchiometrischen Überschuss eingesetzt werden kann.

Dabei können das Phenol und das Phosgen über ein statisches Mischelement vereinigt, in Gegen- oder Gleichstrom beispielsweise über ein Festbett (heterogen) oder in eine oder mehrere Reaktivdestillationskolonnen oder Blasensäulen (homogen) geführt werden, wo das Gemisch zum gewünschten Diarylcarbonat und Chlorwasserstoff abreagiert. Neben Reaktionstürmen, die mit geeigneten Mischelementen versehen sind, können auch Reaktionsbehälter mit Rührvorrichtung eingesetzt werden. Außer statischen Mischelementen können auch spezielle dynamische Mischelemente Anwendung finden. Geeignete statische und dynamische Mischelemente sind aus dem Stand der Technik bekannt.

Nach der Phosgenierung gemäß Schritt (b) erfolgt erfindungsgemäß in Schritt (c) die Abtrennung der bei der Phosgenierung gebildeten Diarylcarbonate. Dies geschieht dadurch, dass zunächst das Reaktionsgemisch der Phosgenierung in einen flüssigen und einen gasförmigen Produktstrom in einer dem Fachmann bekannten Weise aufgetrennt wird. Der flüssige Produktstrom enthält im Wesentlichen das Diarylcarbonat, gegebenenfalls das Lösungsmittel sowie einen geringen Teil an nicht umgesetztem Phosgen. Der gasförmige Produktstrom besteht im Wesentlichen aus Chlorwasserstoffgas, nicht umgesetztes Phosgen, sowie geringfügigen Mengen an gegebenenfalls Lösungsmittel und Inertgasen, wie zum Beispiel Stickstoff und Kohlenmonoxid. Ferner wird der Flüssigstrom gemäß Schritt (c) anschließend einer Aufarbeitung zugeführt, vorzugsweise einer destillativen Aufarbeitung, wobei nacheinander Phosgen sowie gegebenenfalls das Lösungsmittel abgetrennt werden. Gegebenenfalls erfolgt gemäß Schritt (c) außerdem eine weitere Aufarbeitung der gebildeten Diarylcarbonate. Dies geschieht beispielsweise, indem das erhaltene Diarylcarbonat in einer dem Fachmann bekannten Weise gereinigt wird durch Destillation oder Kristallisation.

Die in Schritt d) gegebenenfalls notwendige Reinigung des Chlorwasserstoffs kann dadurch bedingt sein, dass der bei der Reaktion von Phosgen mit einem Phenol erhaltene Chlorwasserstoff im Allgemeinen organische Bestandteile enthält, welche bei der elektrochemischen Oxidation einer wässrigen Chlorwasserstofflösung gemäß Schritt (g) stören können, so dass bei einer Kontamination oder Beschädigung der Ionenaustauschermembran oder des katalytisch aktiven Materials die gesamte Einheit, bestehend aus Ionenaustauschermembran und katalytisch aktivem Material der Elektroden, ausgetauscht werden muss. Zu diesen organischen Bestandteilen zählen beispielsweise Phosgen, das Monophenol oder die gegebenenfalls bei der Diarylcarbonatherstellung eingesetzten Lösungsmittel wie Chlorbenzol, o-Dichlorbenzol oder p-Dichlorbenzol. Erfolgt die Elektrolyse nach dem Membranverfahren, so könnte die Ionenaustauschermembran durch diese organischen Bestandteile oder durch anorganische Verunreinigungen, wie Eisen-, Silizium- oder Aluminium-Verbindungen, in ihrer Funktion beeinträchtigt werden. Die Verunreinigungen können sich auch auf der Ionenaustauschermembran ablagern und dadurch die Spannung der Elektrolyse erhöhen. Wird bei der Elektrolyse eine Gasdiffusionselektrode als Kathode eingesetzt, so kann auch der Katalysator der Gasdiffusionselektrode durch die anorganischen oder organischen Verunreinigungen desaktiviert werden. Darüber hinaus können sich diese Verunreinigungen auf dem Stromkollektor ablagern und dadurch den Kontakt zwischen Gasdiffusionselektrode und Stromkollektor verschlechtern, was einen Spannungsanstieg zur Folge hat. Wird zur Elektrolyse der Salzsäure das Diaphragmaverfahren eingesetzt, so können sich die genannten organischen und anorganischen Bestandteile auf den Graphitelektroden und/oder dem Diaphragma ablagern und dadurch ebenfalls die Elektrolysespannung erhöhen.

Dementsprechend erfolgt in einem weiteren Verfahrensschritt (d) die Abtrennung des bei der Phosgenierung gemäß Schritt (b) erzeugten Chlorwasserstoffs aus dem gasförmigen Produktstrom. Der gasförmige Produktstrom der bei der Abtrennung des Diphenylcarbonats gemäß Schritt (c) erhalten wird, wird gemäß Schritt (d) so behandelt, dass das Phosgen abgetrennt wird und der Chlorwasserstoff als wäßrige Lösung gemäß Schritt (e), gegebenenfalls nach Reinigung gemäß Schritt (f), einer elektrochemischen Oxidation gemäß Schritt (g) zugeführt werden kann.

Die Abtrennung des Chlorwasserstoffs gemäß Schritt (d) erfolgt zunächst, indem Phosgen aus dem gasförmigen Produktstrom abgetrennt wird. Die Abtrennung des Phosgens gelingt durch Verflüssigung von Phosgen, beispielsweise an einem oder mehreren in Reihe geschalteten Kondensatoren. Die Verflüssigung erfolgt vorzugsweise bei Temperaturen im Bereich von -15 bis -40°. Durch diese Tiefkühlung können außerdem gegebenenfalls Teile der organischen Verunreinigungen, wie z.B. Monophenol aus dem gasförmigen Produktstrom entfernt werden.

Zusätzlich oder alternativ kann das Phosgen mit einem kalten Lösungsmittel oder Lösungsmittel-Phosgen-Gemisch aus dem Gasstrom in einer oder mehreren Stufen ausgewaschen werden. Als Lösungsmittel hierfür eignen sich beispielsweise die gegebenenfalls bereits in der Phosgenierung eingesetzten Lösungsmittel Chlorbenzol und o-Dichlorbenzol. Die Temperatur des Lösungsmittels oder Lösungsmittel-Phosgen-Gemisches hierfür liegt im Bereich von -15 bis -46°C.

Das aus dem gasförmigen Produktstrom abgetrennte Phosgen kann wieder der Phosgenierung gemäß Schritt (b) zugeführt werden.

Gegebenenfalls erfolgt anschließend gemäß Schritt (d) eine Reinigung des Chlorwasserstoffs, um den Anteil an organische Verunreinigungen wie z.B. unumgesetztes Monophenol zu verringern. Dies kann beispielsweise mittels Ausfrieren erfolgen, indem in Abhängigkeit von den physikalischen Eigenschaften des Monophenols der Chlorwasserstoff zum Beispiel durch eine oder mehrere Kühlfallen geleitet wird.

In einer bevorzugten Ausführungsform der gemäß Schritt (d) gegebenenfalls vorgesehenen Reinigung des Chlorwasserstoffs werden zwei in Serie geschaltete Wärmetauscher von dem Chlorwasserstoffstrom durchströmt, wobei das abzutrennende Monophenol in Abhängigkeit des Festpunktes zum Beispiel bei -40°C ausgefroren wird. Die Wärmetauscher werden wechselweise betrieben, wobei im jeweils zuerst durchströmten Wärmetauscher der Gasstrom das zuvor ausgefrorene Monophenol auftaut. Das Monophenol kann wieder für die Herstellung des Diarylcarbonats eingesetzt werden. Im nachgeschalteten zweiten Wärmetauscher, der mit einem üblichen Wärmeträgermedium für Kältemaschinen, z.B. eine Verbindung aus der Reihe der Frigene, beaufschlagt ist, wird das Gas unter den Festpunkt des Monophenols abgekühlt, sodass dieses auskristallisiert. Nach abgeschlossenem Auftau- und Kristallisationsvorgang werden der Gasstrom und der Kühlmittelstrom umgeschaltet, sodass sich die Funktion der Wärmetauscher umkehrt. Der chlorwasserstoffhaltige Gasstrom kann auf diese Weise auf vorzugsweise maximal 500 ppm, besonders bevorzugt maximal 50 ppm, ganz besonders bevorzugt auf maximal 20 ppm Monophenolgehalt abgereichert werden.

Alternativ kann die Reinigung des Chlorwasserstoffs in zwei in Serie geschaltete Wärmetauscher gemäß US 6 719 957 erfolgen. Dabei wird der Chlorwasserstoff auf einen Druck von 5 bis 20 bar, bevorzugt 10 bis 15 bar, verdichtet und der komprimierte gasförmige Chlorwasserstoff mit einer Temperatur von 20 bis 60°C, bevorzugt 30 bis 50°C, einem ersten Wärmeaustauscher zugeführt. In diesem wird der Chlorwasserstoff mit einem kalten Chlorwasserstoff einer Temperatur von -10 bis -30°C, der aus einem zweiten Wärmetauscher stammt, gekühlt. Dabei kondensieren organische Bestandteile, die einer Entsorgung oder Wiederverwertung zugeführt werden können. Der in den ersten Wärmetauscher geleitete Chlorwasserstoff verlässt diesen mit einer Temperatur von -20 bis 0°C und wird in dem zweiten Wärmetauscher auf eine Temperatur von - 10 bis -30°C gekühlt. Das im zweiten Wärmetauscher anfallende Kondensat besteht aus weiteren organischen Bestandteilen sowie geringen Mengen Chlorwasserstoff. Zur Vermeidung eines Chlorwasserstoffverlustes wird das aus dem zweiten Wärmetauscher ablaufende Kondensat einer Abtrenn- und Verdampfereinheit zugeführt. Dies kann beispielsweise eine Destillationskolonne sein, in der aus dem Kondensat der Chlorwasserstoff ausgetrieben und in den zweiten Wärmetauscher zurückgeführt wird. Es ist auch möglich, den ausgetriebenen Chlorwasserstoff in den ersten Wärmetauscher zurückzuführen. Der in dem zweiten Wärmetauscher abgekühlte und von organischen Bestandteilen befreite Chlorwasserstoff wird mit einer Temperatur von -10 bis -30°C in den ersten Wärmetauscher geleitet. Nach Erwärmung auf 10 bis 30°C verlässt der von organischen Bestandteilen befreite Chlorwasserstoff den ersten Wärmetauscher.

In einem alternativen Verfahren erfolgt die gemäß Schritt (d) gegebenenfalls vorgesehene Reinigung des Chlorwasserstoffs mittels Adsorption von organischen Verunreinigungen, wie Monophenolreste, an Aktivkohle. Dabei wird beispielsweise der Chlorwasserstoff nach Entfernung von überschüssigem Phosgen bei einem Druck von 0 bis 5 bar, vorzugsweise von 0,2 und 2 bar, über oder durch eine Aktivkohleschüttung geleitet. Die Strömungsgeschwindigkeiten und Verweilzeiten werden dabei in einer dem Fachmann bekannten Weise dem Gehalt an Verunreinigungen angepasst. Die Adsorption von organischen Verunreinigungen ist ebenso an anderen geeigneten Adsorptionsmitteln möglich, so z.B. an Zeolithen.

In einem weiteren alternativen Verfahren kann für die gemäß Schritt (d) gegebenenfalls vorgesehene Reinigung des Chlorwasserstoffs eine Destillation des Chlorwasserstoffes vorgesehen sein. Diese erfolgt nach Kondensation des gasförmigen Chlorwasserstoffes. Bei der Destillation des kondensierten Chlorwasserstoffes wird der gereinigte Chlorwasserstoff als Kopfprodukt der Destillation entnommen, wobei die Destillation unter dem Fachmann bekannte, für eine solche Destillation übliche Bedingungen von Druck, Temperatur u.ä. erfolgt.

Aus dem gemäß Schritt (d) abgetrennten und gegebenenfalls gereinigten Chlorwasserstoff wird danach in Schritt (e) eine wässrige Chlorwasserstofflösung hergestellt. Hierfür wird der Chlorwasserstoff vorzugsweise einer adiabaten Chlorwasserstoffabsorption zugeführt, die in einer Absorptionskolonne unter Zugabe eines geeigneten Absorptionsmittels erfolgt. In einer bevorzugten Ausführungsform besteht das Absorptionsmittel aus einer wässrigen Chlorwasserstofflösung (Salzsäure) im Konzentrationsbereich bis 20 Gew.%, bevorzugt 16 bis 18 Gew.%. Alternativ kann auch eine Salzsäure geringerer Konzentration oder entsalztes Wasser oder ein Dampfkondensat eingesetzt werden. Die adiabate Absorption von Chlorwasserstoff in wässriger Salzsäure zur Herstellung von konzentrierter Salzsäure ist bereits aus dem Stand der Technik bekannt. Die Absorption erfolgt zum Beispiel durch Einleiten des Chlorwasserstoffstroms in den unteren Teil einer Absorptionskolonne, in dem die Absorptionskolonne mit Stoffaustauschelementen, wie zum Beispiel Siebböden oder Packungen, ausgerüstet ist. Auf den oberen Teil der Absorptionskolonne oberhalb der Stoffaustauschelemente wird das Absorptionsmittel aufgegeben. Das Chlorwasserstoffgas wird im Gegenstrom an den Stoffaustauschelementen im Absorptionsmittel absorbiert, d.h. gelöst.

Die optionale Reinigung der wässrigen Salzsäure in Schritt (f) soll organische Verunreinigungen entfernen, die insbesondere bei längerer Betriebszeit der Elektrolyse durch Kontamination oder Beschädigung der Ionenaustauschermembran oder des katalytisch aktiven Materials zu einem Spannungsanstieg führen, was ein Austausch zur Folge haben kann und somit die Wirtschaftlichkeit des Verfahrens negativ beeinflusst. Zur Entfernung organischer Bestandteile wird in WO 02/18675 eine Reinigung der Salzsäure mittels Aktivkohle und gegebenenfalls zusätzlich mittels eines Austauscherharzes, z.B. eines Molekularsiebes, vorgeschlagen.

Der am Kopf der Absorptionskolonne austretende Gasstrom, die Brüden, besteht im prozessüblichen Temperaturbereich von 90 bis 120 °C, bevorzugt von 105 bis 109°C, im Wesentlichen aus Wasserdampf. Daneben sind noch Chlorwasserstoff, Inertgase wie Stickstoff und Kohlenmonoxid sowie noch nicht mit Wasser abreagiertes Phosgen und Restmengen an organische Bestandteile enthalten. Zur Abtrennung kondensierbarer Anteile, wie Wasser, Salzsäure und organische Bestandteile und zur Abführung der Kondensationswärme wird der gasförmige Kopfstrom bevorzugt einer Kondensationseinheit zugeführt. Diese Kondensationseinheit kann zum Beispiel aus einem oder mehreren nacheinander geschalteten kühlwasserbetriebenen Rohrbündelwärmetauschern bestehen. Der flüssige Ablauf dieses Kondensationssystems wird anschließend vorzugsweise einem Abscheider zugeführt, um die auskondensierten organische Anteile von der wässrigen Salzsäurephase abzutrennen. Bei diesem Abscheider handelt es sich vorzugsweise um einen statischen Phasentrenner. Durch entsprechende Trennelemente in diesem Abscheider kann die Trennung von organischer und wässriger Phase unterstützt werden. Die abgeschiedene organische Phase wird einer geeigneten Verwertung zugeführt. Die an organische Verunreinigungen abgereicherte Salzsäurephase kann auf den oberen Teil der Absorptionskolonne rückgeführt werden.

Die im unteren Bereich der Absorptionskolonne ablaufende wässrige Chlorwasserstofflösung (Salzsäure) kann bei Bedarf mit einem dafür geeigneten Kühlaggregat abgekühlt, gegebenenfalls gemäß Schritt (f) gereinigt und anschließend der elektrochemischen Oxidation gemäß Schritt (g) zugeführt werden. Diese im Allgemeinen ca. 24 bis 30 Gew.%-ige, bevorzugt 27 bis 30 Gew.%-ige Salzsäure, im Folgenden auch als aufkonzentrierte Salzsäure bezeichnet, enthält organische Anteile von bevorzugt maximal 0,05 Gew.-%, besonders bevorzugt maximal 0,005 Gew.-%. Der Phosgengehalt der Salzsäure beträgt bevorzugt 0,1 bis 0,0001 Gew.-%, kann jedoch auch weniger als 0,0001 Gew.-% betragen.

Die wässrige Chlorwasserstofflösung wird daher gegebenenfalls in einem Schritt (f) einer Reinigung, insbesondere zur weiteren Verringerung des Phosgengehalts und gebenenfalls des Anteils an organische Bestandteile, unterworfen. Dies kann mittels Strippung in einer Kolonne in einer dem Fachmann bekannten Weise geschehen, z.B. durch Einleiten der aufkonzentrierten Salzsäure in eine Packungskolonne, die entweder mit einem Umlaufverdampfer oder mit einer Dampfeinspeisung versehen ist. Während die Brüden der Stripperkolonne zurück in die Absorptionskolonne geführt werden können, kann der flüssige Austrag der Kolonne als gereinigte aufkonzentrierte Salzsäure, gegebenenfalls über einen Kühler, der Salzsäure-Elektrolyse gemäß Schritt (g) zugeführt werden. Anstatt die Strippung in einer separaten Stripperkolonne durchzuführen, kann sie auch in der Absorptionskolonne selbst durch Direkteinspeisung von Dampf, bevorzugt in den unter der Absorptionskolonne angeordneten Abtriebsteil, erfolgen. Anstelle der Strippung in der Absorptionskolonne kann auch durch partielles Destillieren mit Hilfe eines der Absorptionskolonne nachgeschalteten Wärmetauschers der Gehalt an organische Verunreinigungen im Chlorwasserstoff reduziert werden.

Darüber hinaus wird die wässrige Chlorwasserstofflösung in dem gebenenfalls vorgesehenen Schritt (f) einer Reinigung zur Entfernung von Eisen-, Aluminium- und/oder Siliziumverbindungen unterworfen. Bevorzugt erfolgt die Entfernung von Eisen-, Aluminium- und/oder Siliziumverbindungen mittels chelatbildender Ionenaustauscher. Derartige Ionenaustauscher sind kommerziell erhältlich.

So kann beispielsweise die Entfernung von Eisen-Verbindungen durch Ionenaustauscher vom Typ Amberjet 4400CI der Fa. Rohm & Haas oder Lewatit M500 der Fa. LANXESS erfolgen. Bevorzugt beträgt die Konzentration der Salzsäure zur Entfernung von Eisen mindestens 8 Gew.-%.

Zur Entfernung eisenhaltiger Verbindungen kann auch eine Fällung als schwerlösliche Verbindungen und eine anschließende Filtration vorgesehen sein.

Der wässrigen Chlorwasserstoff-Lösung vor der elektrochemischen Oxidation gemäß Schritt (g) können Metallionen aus der Gruppe der Platinmetalle, vorzugsweise Platin und / oder Palladium zugesetzt werden.

Nach Herstellung einer wässrigen Chlorwasserstofflösung gemäß Schritt (e) sowie gegebenenfalls nach einer Reinigung der wässrigen Chlorwasserstofflösung gemäß Schritt (f) wird die Salzsäure einer Elektrolysezelle zugeführt. Die elektrochemische Oxidation der Salzsäure gemäß Schritt (g) erfolgt nach dem Membranverfahren.

Gemäß WO 97/24320 sind u.a. sogenannte Festelektrolytsysteme, z.B. Nafion^{®}-Membrane, eingesetzt werden, wobei die Kathode auf einer Seite der Ionenaustauschermembran anliegt. Die Kathode ist z.B. eine Gasdiffusionselektrode. Das katalytisch aktive Material der Kathode ist in die Ionenaustauschermembran eingearbeitet oder kann auf die Ionenaustauschermembran aufgebracht sein.

Die elektrochemische Oxidation einer wässrigen Lösung von Chlorwasserstoff unter Verwendung einer Gasdiffusionselektrode als Kathode ist z.B. in WO 00/73538 und WO 02/18675 beschrieben. Dabei wird als Katalysator für die Sauerstoffreduktion an der Kathode Rhodiumsulfid eingesetzt. Gemäß WO 02/18675 ist dieser Katalysator weitgehend beständig gegen organische Bestandteile, die als Verunreinigungen in der Salzsäure vorliegen können und z.B. aus vorgeschalteten Syntheseschritten stammen. Die organischen Bestandteile gelangen über die Ionenaustauschermembran aus dem Anodenraum in den Kathodenraum.

Die elektrochemische Oxidation der Salzsäure gemäß Schritt (g) kann nach dem Membranverfahren in einer Zweiraum-Elektrolysezelle, bestehend aus Anodenraum und Kathodenraum, oder in einer Dreiraum-Elektrolyszelle, bestehend aus Anodenraum, Kathodenraum und einem Elektrolytraum zwischen Anoden- und Kathodenraum, durchgeführt werden. Bevorzugt wird eine Zweiraum-Elektrolysezelle gewählt. Beim Membranverfahren ist der Anodenraum von dem Kathodenraum durch eine Ionenaustauschermembran (nachfolgend vereinfacht auch als Membran bezeichnet), insbesondere eine Kationenaustauschermembran, getrennt. Der Abstand der Elektroden (Anode und Kathode) von der Membran beträgt vorzugsweise 0 bis 3 mm, beson-ders bevorzugt 0 bis 2 mm. Geeignete Ionenaustauschermembrane sind kommerziell erhältlich, wie z.B. einschichtige Ionenaustauschermembrane mit Sulfonsäuregruppen. Beispielsweise kann eine Membran vom der Fa. DuPont Typ Nafion^{®} 117 eingesetzt werden.

Bei der Elektrolyse von Salzsäure nach dem Membranverfahren können Elektroden eingesetzt werden, die Graphit enthalten, wobei die Anode aus Graphit bestehen kann.

In einer bevorzugten Ausführungsform erfolgt die elektrochemische Oxidation der wässrigen Lösung von Chlorwasserstoff gemäß Schritt (g) nach dem Membranverfahren mit einer Gasdiffusionselektrode als Sauerstoffverzehrkathode. Dabei kann die Elektrolysezelle sowohl aus zwei Kammern als auch aus drei Kammern, bevorzugt jedoch aus zwei Kammern, bestehen. Der Kathodenhalbzelle wird ein sauerstoffhaltiges Gas, z.B. Sauerstoff, Luft oder mit Sauerstoff angereicherte Luft, zugeführt. Der Sauerstoff wird an der Gasdiffusionselektrode reduziert, wobei Wasser gebildet wird. Der Anodenhalbzelle wird die wässrige Chlorwasserstoff-Lösung zugeführt, wobei der Chlorwasserstoff an der Anode zu Chlor oxidiert wird. Die Anodenhalbzelle und die Kathodenhalbzelle sind durch eine Kationenaustauschermembran voneinander getrennt. Die Elektrolyse von Salzsäure unter Verwendung einer Gasdiffusionselektrode als Kathode ist z.B. in WO 00/73538 beschrieben.

Die Elektrolysezelle kann entweder aus einem nichtmetallischen Werkstoff gemäß DE 103 47 703 A oder aus einem metallischen Werkstoff bestehen. Als metallischer Werkstoff für die Elektrolysezelle eignet sich z.B. Titan oder eine Titan-Legierung, wie eine Titan-Palladium-Legierung. Dabei sind die Halbschalen für die Anode- und Kathodenhalbzelle, der Stromverteiler und die Stromzuführungen aus Titan oder einer Titan-Legierung gefertigt.

Die Anode kann z.B. gemäß DE 102 34 806 A ausgeführt sein. Dabei besteht die Anode aus Metall, vorzugsweise aus Titan mit einer Beschichtung aus Edelmetalloxid, z.B. aus Rutheniumoxid. Weiterhin kann die Anode aus Titan gemäß DE 102 00 072 A eine Zwischenschicht aus Titancarbid oder Titanborid aufweisen, welche mittels Plasma- oder Flammspritzen auf die Titananode aufgebracht wird, bevor die Beschichtung aus einem Edelmetalloxid aufgebracht wird. Gemäß DE 102 34 806 A weist die Metallanode Öffnungen für den Durchlass des während der Elektrolyse gebildeten Gases auf, wobei die Öffnungen bevorzugt Leitstrukturen aufweisen, welche das gebildete Gas auf die der Ionenaustauschermembran abgewandten Seite der Metallanode ableiten. Dabei sollte die Gesamtquerschnittsfläche der Öffnungen im Bereich von 20% bis 70% der Fläche betragen, welche durch die Höhe und Breite der Anode gebildet wird. Die Metallanode kann außerdem einen wellenförmigen, zickzackförmigen oder rechteckförmigen Querschnitt aufweisen. Die Tiefe der Anode sollte dabei mindestens 1 mm betragen. Das Verhältnis von elektrochemisch aktiver Fläche der Metallanode zu der Fläche, welche durch die Höhe und Breite der Metallelektrode gebildet wird, sollte mindestens 1,2 betragen. In einer speziellen Ausführungsform kann die Metallanode aus zwei aneinander liegenden Streckmetallen bestehen, wobei das zur Ionenaustauschermembran weisende Streckmetall feiner strukturiert ist als das von der Ionenaustauschermembran abweisende Streckmetall. Dabei ist ferner das feiner strukturierte Streckmetall flach gewalzt und das gröber strukturierte Streckmetall so angeordnet, dass die Maschenstege in Richtung der Kathode geneigt sind und als Leitstrukturen dienen. Alternativ kann die Anode auch aus einem Streckmetall bestehen. Grundsätzlich sollte die Anode eine freie Fläche von 15 bis 70 % aufweisen. Die Dicke der Streckmetalle ist so zu wählen, dass kein zusätzlicher elektrischer Widerstand bei einer bipolaren Verschaltung der einzelnen Elektrolysezellen (Zellelemente) zu einem Elektrolyseur auftritt. Der elektrische Widerstand hängt im Wesentlichen von der elektrischen Kontaktierung der Anode ab, wie zum Beispiel Anzahl der stromzuführenden Verbindungselemente zwischen Anode und Rückwand der Anodenhalbzelle.

Bei der Elektrolyse mittels Gasdiffusionselektrode können der Anodenraum und der Kathodenraum von einer handelsüblichen Ionenaustauschermembran getrennt sein. Beispielsweise können Ionenaustauschermembranen der Fa. DuPont vom Typ Nafion^{®} 324 oder Nafion^{®} 117 eingesetzt werden. Bevorzugt wird eine Membran eingesetzt, welche, wie in WO 05/12596 beschrieben, auf der zur Gasdiffusionselektrode gewandten Seite eine glatte Oberflächenstruktur aufweist. Die glatte Oberflächenstruktur der Membran erlaubt es, dass die Gasdiffusionselektrode und die Membran derart aneinander anliegen, dass unter einem Druck von 250 g/cm² und einer Temperatur von 60 °C die Kontaktfläche wenigstens 50 % der geometrischen Fläche der Membran beträgt.

Der kathodische Stromverteiler, auf den die Gasdiffusionselektrode aufgebracht wird, ist bevorzugt gemäß DE 102 03 689 A ausgeführt. Dieser weist eine freie Fläche von weniger als 65 %, jedoch von mehr als 5 % auf. Die Dicke des Stromverteilers beträgt mindestens 0,3 mm. Er kann aus einem Streckmetall, Netz, Gewebe, Schaum, Vlies, Schlitzblech oder Lochplatte aus Metall bestehen. Vorzugsweise besteht der kathodische Stromverteiler aus einem Streckmetall mit einer Maschenlänge von 4 bis 8 mm, einer Maschenbreite 3 bis 5 mm, einer Stegbreite von 0,4 bis 1,8 mm und einer Dicke von 0,4 bis 2 mm. Zusätzlich kann der kathodische Stromverteiler ein zweites Streckmetall als Träger für das erste Streckmetall aufweisen. Das zweite Streckmetall als Träger hat bevorzugt eine Maschenlänge von 10 bis 40 mm, eine Maschenbreite von 5 bis 15 mm, eine Stegbreite von 2 bis 5 mm sowie eine Dicke von 0,8 bis 4 mm. Als Träger kann auch ein Netz eingesetzt werden, welches vorzugsweise eine Drahtdicke von 1 bis 4 mm und eine Maschenweite von 7 bis 25 mm besitzt. Weiterhin kann als Träger ein Lochblech oder Schlitzblech eingesetzt werden, welches bevorzugt eine offene Fläche von weniger als 60 % und eine Dicke von 1 bis 4 mm besitzt. Als Werkstoff für den kathodischen Stromverteiler kann z.B. Titan oder eine edelmetallhaltige Titanlegierung, wie z.B. Titan-Palladium eingesetzt werden. Ist der Stromverteiler ein Streckmetall, so ist dieses bevorzugt gewalzt.

Als Gasdiffusionselektrode kann eine handelsübliche Gasdiffusionselektrode eingesetzt werden, die mit einem geeigneten Katalysator ausgerüstet ist. Geeignete Katalysatoren enthalten gemäß WO 00/73538 Rhodium und/oder wenigstens ein Rhodiumsulfid oder eine Mischung aus Rhodium und wenigstens einem Rhodiumsulfid. Gemäß EP 931 857 A können außerdem Rhodium und/oder Rhodiumoxid oder deren Mischungen eingesetzt werden. Die Gasdiffusionselektrode besteht bevorzugt aus einem elektrisch leitfähigen Gewebe, Papier oder Vlies aus Kohlenstoff, wobei das Gewebe, Papier oder Vlies auf einer Seite mit einer kohlenstoffhaltigen Katalysatorschicht und auf der anderen Seite mit einer Gasdiffusionsschicht versehen ist. Der Katalysator wird bevorzugt auf einen Träger, vorzugsweise aus Kohlenstoff, aufgebracht, wobei Polytetrafluorethylen-Teilchen integriert sind, die an die Trägerstruktur gekoppelt sind. Die Gasdiffusionsschicht besteht vorzugsweise aus Kohlenstoff und Polytetrafluorethylen-Teilchen, bei der beispielsweise das Verhältnis von Kohlenstoff zu PTFE 50:50 beträgt. Die Gasdiffusionselektrode kann beispielsweise so angeordnet werden, dass sie nicht fest mit der Ionenaustauschermembran verbunden ist. Die Kontaktierung der Gasdiffusionselektrode mit dem Stromverteiler und der Ionenaustauschermembran erfolgt bevorzugt durch Presskontakt, d.h. die Gasdiffusionselektrode, der Stromverteiler und die Membran werden aneinander gepresst. Die Gasdiffusionselektrode kann mit dem Stromkollektor gemäß DE 101 48 600 A verbunden sein.

Die Elektrolyse von Salzsäure nach dem Membranverfahren mit Gasdiffusionselektrode wird üblicherweise bei einer Temperatur von 40 bis 70°C durchgeführt. Die Konzentration der wässrigen Lösung von Chlorwasserstoff im Anodenraum beträgt 10 bis 20 Gew.%, bevorzugt 12 bis 17 Gew.%. Die Zelle kann beispielsweise so betrieben werden, dass der Druck im Anodenraum höher ist als der Druck im Kathodenraum. Dadurch wird die Kationenaustauschermembran auf die Gasdiffusionselektrode und diese wiederum auf den Stromverteiler gedrückt. Alternativ kann eine Konstruktion der Elektrolysezelle gewählt werden, die in DE 101 38 214 A beschrieben wird. Die Anode und/oder der Stromverteiler sind elastisch gelagert, beispielsweise indem sie mittels Federn mit der Rückwand der jeweiligen Halbzelle verbunden sind. Beim Zusammenbau der Zelle entsteht eine sogenannte zero gap Anordnung, bei der die Anode in direktem Kontakt mit der Ionenaustauschermembran, diese wiederum in direktem Kontakt mit der Gasdiffusionselektrode und diese wiederum in direktem Kontakt mit dem Stromverteiler steht. Die elastische Lagerung bewirkt das Aneinanderpressen von Anode, Membran, Gasdiffusionselektrode und Stromverteiler.

In einer bevorzugten Ausführungsform des Elektrolyseverfahrens wird beim Anfahren der Elektrolysezelle gemäß DE 10 152 275 A das Anodenhalbelement wird mit einer 5 bis 20 Gew.%-igen Salzsäure gefüllt, wobei die Salzsäure mindestens 10 ppm freies Chlor enthält und die Konzentration der Salzsäure während der Inbetriebnahme mehr als 5 Gew.-% beträgt. Der Volumenstrom der Salzsäure durch den Anodenraum wird so eingestellt, dass zu Beginn der Elektrolyse die Salzsäure mit einer Geschwindigkeit im Anodenraum von 0,05 bis 0,15 cm/s strömt. Die Elektrolyse wird mit einer Stromdichte von 0,5 bis 2 kA/m² gestartet und in Zeitintervallen von 5 bis 25 Minuten um jeweils 0,5 bis 1,5 kA/m² erhöht. Nachdem eine vorgegebene Stromdichte von vorzugsweise 4 bis 7 kA/m² erreicht ist, wird der Volumenstrom der Salzsäure so eingestellt, dass die Salzsäure im Anodenhalbelement mit einer Geschwindigkeit von 0,2 bis 0,4 cm/s strömt.

Eine besonders vorteilhafte Betriebsweise der Elektrolysezelle kann gemäß DE 101 38 215 A erfolgen, wonach die Elektrolysezelle zur Erniedrigung der Zellspannung mit einem erhöhten Druck in der Kathodenkammer betrieben wird. Der Differenzdruck zwischen Anodenraum und Kathodenraum sollte 0,01 bis 1000 mbar und der Sauerstoffdruck im Kathodenraum mindestens 1,05 bar absolut betragen.

Erfindungsgemäß wird im nächsten Verfahrensschritt (h) wenigstens ein Teil des gemäß Schritt (g) hergestellten Chlors in die Herstellung von Phosgen gemäß Schritt (a) rückgeführt. Vor der Rückführung wird das Chlor vorzugsweise in einer ein- oder mehrstufigen Kühlung mittels eines Kühlaggregats, z.B. eines Röhrenwärmetauscher, abgekühlt und getrocknet. Die Trocknung kann zum Beispiel mit Hilfe eines geeigneten Trocknungsmittels in einer mit Stoffaustauschelementen bestückten Absorptionskolonne erfolgen. Ein geeignetes Trocknungsmittel kann, wie z.B. in DE 10 235 476 A beschrieben, neben Molsieben oder hygroskopischen Adsorbentien z.B. Schwefelsäure sein. Die Trocknung kann ein- oder mehrstufig erfolgen. Bevorzugt erfolgt die Trocknung zweistufig, indem das zu trocknende Chlor in einer ersten Stufe mit einer Schwefelsäure geringerer Konzentration, vorzugsweise 70 bis 80%, besonders bevorzugt 75 bis 80%, in Kontakt gebracht wird. In einer zweiten Stufe wird die Restfeuchte mittels einer höher konzentrierten Schwefelsäure von bevorzugt 88 bis 96%, besonders bevorzugt 92-96%, aus dem Chlor entfernt. Das auf diese Art getrocknete Chlor mit einer Restfeuchte von bevorzugt maximal 100 ppm, besonders bevorzugt maximal 20 ppm kann durch einen Tröpfchenabscheider geleitet werden, um gegebenenfalls darin noch enthaltene Schwefelsäuretröpfchen zu entfernen.

Die Kreislauffahrweise des erfindungsgemäßen Verfahrens erfordert es, neben dem gemäß Schritt (g) mittels Elektrolyse hergestellten Chlors für die Phosgenherstellung gemäß Schritt (a) eine weitere Teilmenge Chlor bereitzustellen, da Verluste an Chlor und Chlorwasserstoff in dem Chlor-Chlorwasserstoff-Kreislauf auftreten. Die Bereitstellung einer weiteren Teilmenge Chlor kann in Form von elementarem Chlor aus einer externen Quelle, beispielsweise der Elektrolyse einer wässrigen Natriumchloridlösung, stammen. Die auftretenden Verluste von Chlor und Chlorwasserstoff können aber auch dadurch ausgeglichen werden, dass eine Teilmenge Chlorwasserstoff aus einer externen Quelle bereitgestellt wird, z.B. aus einer Isocyanatproduktionsanlage (e.g. MDI, TDI), bei dem Chlorwasserstoff als gasförmiges Nebenprodukt anfällt. Eine Teilmenge Chlorwasserstoff in Form einer wässrigen Chlorwasserstofflösung aus einer externen Quelle, z.B. aus einem Produktionsverfahren, bei dem eine wässrige Chlorwasserstofflösung als Nebenprodukt anfällt, wird vorzugsweise als ca 30 Gew.%-ige Salzsäure einer Elektrolyse zugeführt. Eine Salzsäure geringerer Konzentration kann alternativ der Absorption von Chlorwasserstoff gemäß Schritt (e) zugeführt werden.

Das Phosgen kann wieder in die Herstellung des Diarylcarbonat gemaß Schritt b) eingesetzt werden.

Durch Umesterung des gemäß Schritt (b) hergestellten Diarylcarbonats mit wenigstens einem Bisphenol ergibt ein Oligo-/Polycarbonat und Monophenol, das wiederum in Schritt b) eingesetzt werden kann.

Für das erfindungsgernäße Verfahren geeignete Bisphenole sind Dihydroxydiarylalkane der Formel (II),

HO-Z-OH (II)

worin Z ein divalenter organischer Rest mit 6 bis 30 Kohlenstoffatomen ist, der eine oder mehrere aromatische Gruppen enthält. Beispiele solcher Verbindungen, die in Schritt a) des erfindungsgemäßen Verfahrens eingesetzt werden können sind Dihydroxydiarylalkane wie Hydrochinon, Resorcin, Dihydroxydiphenyl, Bis-(hydroxyphenyl)-alkane, Bis(hydroxyphenyl)-cycloalkane, Bis-(hydroxyphenyl)-sulfide, Bis-(hydroxyphenyl)-ether, Bis-(hydroxyphenyl)-ketone, Bis-(hydroxyphenyl)-sulfone, Bis-(hydroxyphenyl)-sulfoxide, a,a'-Bis-(hydroxyphenyl)-diisopropylbenzole, so wie deren alkylierte, kernalkylierte und kernhalogenierte Verbindungen.

Bevorzugte Bisphenole sind 4,4'-Dihydroxydiphenyl, 2,2-Bis-(4-hydroxyphenyl)-1-phenylpropan, 1,1-Bis-(4-hydroxyphenyl)-phenylethan, 2,2-Bis-(4-hydroxyphenyl)-propan (Bisphenol A (BPA)), 2,4-Bis-(4-hydroxyphenyl)-2-methylbutan, 1,3-Bis-[2-(4-hydroxyphenyl)-2-propyl]-benzol (Bisphenol M), 2,2-Bis-(3-methyl-4-hydroxyphenyl)-propan, Bis-(3,5-dimethyl-4-hydroxyphenyl)-methan, 2,2-Bis-(3,5-dimethyl-4-hydroxyphenyl)-propan, Bis-(3,5-dimethyl-4-hydroxyphenyl)-sulfon, 2,4-Bis-(3,5-dimethyl-4-hydroxyphenyl)-2-methylbutan, 1,3-Bis-[2-(3,5-dimethyl-4-hydroxyphenyl)-2-propyl]-benzol, 1,1-Bis-(4-hydroxyphenyl)-cyclohexan und 1,1-Bis-(4-hydroxyphenyl)-3,3,5-trimethyl-cyclohexan (Bisphenol TMC).

Besonders bevorzugte Bisphenole sind 4,4'-Dihydroxydiphenyl, 1,1-Bis-(4-Hydroxyphenyl)-phenylethan, 2,2-Bis-(4-hydroxyphenyl)-propan (Bisphenol A (BPA)), 2,2-Bis(3,5-dimethyl-4-hydroxyphenyl)-propan, 1,1-Bis-(4-hydroxyphenyl)-cyclohexan und 1,1-Bis-(4-hydroxyphenyl)-3,3,5-trimethylcyclohexan (Bisphenol TMC).

Geeignete Bisphenole sind z.B. in den US 2 999 835 A, US 3 148 172 A, US 2 991 273 A, US 3 271 366 A, US 4 982 014 A und US 2 999 846 A in den deutschen Offenlegungsschriften DE 15 70 703 A, DE 20 63 050 A, DE 20 36 052 A, DE 22 11 956 A und DE 38 32 396 A, der französischen Patentschrift FR 1 561 518 A, in der Monographie von H. Schnell, Chemistry and Physics of Polycarbonates, Interscience Publishers, New York 1964, S. 28ff; S.102ff und von D.G. Legrand, J.T. Bendler, Handbook of Polycarbonate Science and Technology, Marcel Dekker New York 2000, S. 72ff. beschrieben.

Im Falle der erfindungsgemäßen Herstellung von Homopolycarbonaten wird nur ein Bisphenol eingesetzt, im Falle der erfindungsgemäßen Herstellung von Copolycarbonaten werden mehrere Bisphenole eingesetzt, wobei selbstverständlich die verwendeten Bisphenole, wie auch alle anderen der Synthese zugesetzten Chemikalien und Hilfsstoffe mit den aus ihrer eigenen Synthese, Handhabung und Lagerung stammenden Verunreinigungen kontaminiert sein können, obwohl es wünschenswert ist, mit möglichst sauberen Rohstoffen zu arbeiten.

Es sei hier betont, dass das erfindungsgemäße Verfahren praktisch für alle bekannten Bisphenole eingesetzt werden kann.

Die Polycarbonate können durch den Einsatz geringer Mengen Kettenabbrecher und Verzweiger bewusst und kontrolliert modifiziert werden. Geeignete Kettenabbrecher und Verzweiger sind literaturbekannt. Einige sind beispielsweise in der DE-A 38 33 953 beschrieben. Bevorzugt eingesetzte Kettenabbrecher sind Phenol oder Alkylphenole, insbesondere Phenol, p-tert.Butylphenol, iso-Octylphenol, Cumylphenol, deren Chlorkohlensäureester oder Säurechloride von Monocarbonsäuren bzw. Gemischen aus diesen Kettenabbrechern. Bevorzugte Kettenabbrecher sind Phenol, Cumylphenol, Isooctylphenol und para-tert.-Butylphenol.

Beispiele für als Verzweiger geeignete Verbindungen sind aromatische oder aliphatische Verbindungen mit mehr als drei, bevorzugt drei oder vier Hydroxygruppen. Besonders geeignete Beispiele mit drei oder mehr als drei phenolischen Hydroxylgruppen sind Phloroglucin, 4,6-Dimethyl-2,4,6-tri-(4-hydroxyphenyl)-hepten-2, 4,6-Dimethyl-2,4,6-tri-(4-hydroxyphenyl)-heptan, 1,3,5-Tri-(4-hydroxyphenyl)-benzol, 1,1,1-Tri-(4-hydroxyphenyl)-ethan, Tri-(4-hydroxyphenyl)-phenylmethan, 2,2-Bis-(4,4-bis-(4-hydroxyphenyl)-cyclohexyl]-propan, 2,4-Bis-(4-hydroxyphenyl-isopropyl)-phenol und Tetra-(4-hydroxyphenyl)-methan.

Beispiele für sonstige als Verzweiger geeignete trifunktionelle Verbindungen sind 2,4-Dihydroxybenzoesäure, Trimesinsäure, Cyanurchlorid und 3,3-Bis-(3-methyl-4-hydroxyphenyl)-2-oxo-2,3-dihydroindol.

Besonders bevorzugte Verzweiger sind 3,3-Bis-(3-methyl-4-hydroxyphenyl)-2-oxo-2,3-dihydroindol und 1,1,1-Tri-(4-hydroxyphenyl)-ethan.

Die gegebenenfalls mitzuverwendenden 0,05 bis 2 Mol-%, bezogen auf eingesetzte Bisphenole, an Verzweigern, können mit den Bisphenolen zusammen eingesetzt werden.

Es ist darauf zu achten, dass die Reaktionskomponenten für den ersten Schritt, die Umesterung, also, die Bisphenole und die Diarylcarbonate frei von Alkali und Erdalkaliionen sind, wobei Mengen von kleiner 0,1 ppm an Alkali- und Erdalkaliionen toleriert werden können. Derart reine Bisphenole bzw. Diarylcarbonate sind erhältlich, indem man die Bisphenole bzw. Diarylcarbonate umkristallisiert, wäscht oder destilliert. Beim erfindungsgemäßen Verfahren soll der Gehalt an Alkali- und Erdalka-limetallionen sowohl im Bisphenol als auch im Diarylcarbonate einen Wert von < 0,1 ppm betragen.

Die Umesterungsreaktion des Bisphenols und des Diarylcarbonats in der Schmelze wird bevorzugt in zwei Stufen durchgeführt. In der ersten Stufe findet das Aufschmelzen des Bisphenols und des Diarylcarbonats bei Temperaturen von 80 - 250°C, bevorzugt 100 - 230°C, besonders bevorzugt 120 -190°C unter normalem Druck in 0-5 Stunden, bevorzugt 0,25 - 3 Stunden statt. Nach Zugabe des Katalysators wird durch Anlegen von Vakuum (bis zu 2 mm Hg) und Erhöhung der Temperatur (auf bis zu 260°C) durch Abdestillieren des Monophenols das Oligocarbonat aus dem Bisphenol und dem Diarylcarbonat hergestellt. Das so hergestellte Oligocarbonat hat eine mittlere Gewichtsmol-masse M_{w} (ermittelt durch Messung der rel. Lösungsviskosität in Dichlormethan oder in Mischungen gleicher Gewichtsmengen Phenol / o-Dichlorbenzol, geeicht durch Lichtstreuung) im Bereich von 2000 bis 18 000 bevorzugt von 4 000 bis 15 000. Hierbei wird die Hauptmenge an Monophenol (80 %) aus dem Prozeß wiedergewonnen.

In der zweiten Stufe wird bei der Polykondensation durch weiteres Erhöhen der Temperatur auf 250 - 320°C, bevorzugt 270 - 295°C und einem Druck von < 2 mm Hg das Polycarbonat hergestellt. Hierbei wird der Rest an Monophenolen zurückgewonnen. Es können geringe Verluste an Monophenolen < 5 %, bevorzugt < 2 %, besonders bevorzugt < 1 % auftreten, verursacht durch Endgruppen im Polycarbonat und Restmonophenol im Polycarbonat. Diese Verluste müssen durch entsprechende Mengen an Monophenol für die Herstellung des Diarylcarbonats ausgeglichen werden.

Katalysatoren im Sinne des erfindungsgemäßen Verfahren für die Umesterung des Bisphenols und des Diarylcarbonats zum Polycarbonat sind alle anorganische oder organischen basischen Verbindungen beispielsweise Lithium-, Natrium-, Kalium-, Cäsium-, Calzium-, Barium-, Magnesium-, - hydroxide, -carbonate, -halogenide, -phenolate, -diphenolate, -fluoride, -acetate, -phosphate, - hydrogenphosphate, -boranate, Stickstoff- und Phosphorbasen wie beispielsweise Tetramethylammoniumhydroxid, Tetramethylammoniumacetat, Tetramethylammoniumfluorid, Tetramethylammoniumtetraphenylboranat, Tetraphenylphosphoniumfluorid, Tetra-phenylphosphoniumtetraphenylboranat, Dimethyldiphenylammoniumhydoxid, Tetraethylammoniumhydroxid, DBU, DBN oder Guanidinsysteme wie beispielsweise das 1,5,7-Triazabicyclo-[4,4,0]-dec-5-en, 7-Phenyl-1,5,7-triazabicyclo-[4,4,0]-dec-5-en, 7-Methyl-1,5,7-tria-zabicyclo-[4,4,0]-dec-5-en, 7,7'-Hexyli-dendi-1,5,7-triazabi-cyclo-[4,4,0]-dec-5-en, 7,7'-Decyliden-di-1,5,7-triazabicyclo-[4,4,0]-dec-5-en, 7,7'-Dodecyliden-di-1,5,7-tri-aza-bicyclo-[4,4,0]-dec-5-en oder Phosphazene wie beispielsweise die Phosphazen-Base P₁-t-Oct = tert.-Octyl-imino-tris-(dimethylamino)-phosphoran, Phosphazen-Base P₁-t-Butyl = tert.-Butyl-imino-tris-(dimethylamino)-phosphoran, BEMP = 2-tert.-Butylimino-2-diethylamino-1,3-dimethyl-perhydro-1,3,2-diaza-2-phosphoran.

Diese Katalysatoren werden in Mengen von 10⁻² bis 10⁻⁸ Mol, bezogen auf 1 Mol Bisphenol, eingesetzt.

Die Katalysatoren können auch in Kombination (zwei oder mehrere) miteinander eingesetzt werden.

Beim Einsatz von Alkali-/Erdalkali-Metallkatalysatoren kann es vorteilhaft sein, die Alkali-/ Erdalkali-Metallkatalysatoren zu einem späteren Zeitpunkt (z.B. nach der Oligocarbonatsynthese bei der Polykondensation in der zweiten Stufe) zuzusetzen. Die Zugabe des Alkali-/Erdalkali-Metallkatalysators kann z.B. als Feststoff oder als Lösung in Wasser, Phenol, Oligocarbonat oder Polycarbonat erfolgen.

Die Mitverwendung von Alkali- bzw. Erdalkali-Metallkatalysatoren widerspricht nicht der vorstehend erwähnten Forderung nach Reinheit der Reaktionspartner.

Die Reaktion des Bisphenols und des Diarylcarbonats zum Polycarbonat kann im Sinne des erfindungsgemäßen Verfahrens diskontinuierlich oder bevorzugt kontinuierlich durchgeführt werden, beispielsweise in Rührkesseln, Dünnschichtverdampfern, Fallfilmverdampfern, Rührkesselkaskaden, Extrudern, Knetern, einfachen Scheibenreaktoren und Hochviskosscheibenreaktoren.

Die aromatischen Polycarbonate des erfindungsgemaßen Verfahrens sollen mittlere Gewichtsmolmassen M_{w} von 18000 bis 80000 vorzugsweise 19000 bis 50000 haben, ermittelt durch Messung der rel. Lösungsviskosität in Dichlormethan oder in Mischungen gleicher Gewichtsmengen Phenol / o-Dichlorbenzol, geeicht durch Lichtstreuung.

Es ist vorteilhaft, die aus der Umesterung des Bisphenols und des Diarylcarbonats zum Polycarbonat abgespaltenen und isolierten Monophenole vor dem Einsatz in die Diarylcarbonatsynthese aufzureinigen. Die Rohmonophenole, die beim Umesterungsprozeß isoliert werden, können je nach Umesterungsbedingungen und Destillationsbedingungen u.a. mit Diarylcarbonaten, dem Bisphenol, Salicylsäure, Isopropenylphenol, Phenoxybenzoesäurephenylester, Xanthon, dem Hydroxymonoarylcarbonat verunreinigt sein. Die Reinigung kann nach den üblichen Reinigungsverfahren, also z. B. Destillation oder Umkristallisation erfolgen. Die Reinheit der Monophenole liegt dann bei > 99 %, bevorzugt bei > 99,8 %, besonders bevorzugt bei > 99,95 %.

Die Vorteile des erfindungsgemäßen integrierten Verfahrens zur Herstellung von Diarylcarbonaten und Polycarbonaten unter elektrochemischer Oxidation einer wässrigen Lösung des bei der Diarylcarbonatherstellung anfallenden Chlorwasserstoffs zur Rückgewinnung von Chlor für die Synthese von Phosgen sind der einfachere Betrieb in Vergleich mit einer katalytischen Oxidation nach dem Deacon-Verfahren und die höhere Stromausbeute unter Vermeidung der Wasserstoffproduktion in Vergleich mit einer klassischen Elektrolyse.

Die Herstellung von Diarylcarbonaten und Polycarbonaten im Verbund mit der elektrochemischen Oxidation von Salzsäure bietet durch die Erzeugung einer aufkonzentrierten Salzsäure von rund 30% aus einer Salzsäure von rund 17% in Schritt e) zusätzlich die Möglichkeit, bei Bedarf aufkonzentrierte Salzsäure für andere Anwendungen dem Kreislauf zu entnehmen. Eine mögliche Verwendung dieser aufkonzentrierten Salzsäure liegt im Nahrungsmittelbereich. Hierfür kann für die nach dem erfindungsgemäßen Verfahren hergestellte aufkonzentrierte Salzsäure z.B. durch absorptive Nachreinigung an einem Aktivkohlebett, wie sie aus dem Stand der Technik bekannt ist, eine für die Nahrungsmittelindustrie ausreichend hohe Reinheit erreicht werden.

Das erfindungsgemäße Verfahrens zur Herstellung von Diarylcarbonaten und Polycarbonaten kann gegebenenfalls mit anderen Einsatzmöglichkeiten des bei der Diarylcarbonatherstellung anfallenden Chlorwasserstoffs wie z. B. die Verwendung als Edukt für die Ethylendichloridherstellung bei der PVC-Produktion kombiniert werden.

Die Beispiele sollen das erfindungsgemäße Verfahren anhand der Herstellung von Diphenylcarbonat und elektrochemische Oxidation des dabei anfallenden Chlorwasserstoffs zu Chlor für die Synthese von Phosgen, das wieder für das Diphenylcarbonat-Herstellungsverfahren verwendet werden kann, darstellen. Das bei der Polycarbonatherstellung anfallende Phenol wird in die Herstellung von Diphenylcarbonat rückgeführt.
**Fig. 1** zeigt ein integriertes Verfahren zur Herstellung von Polycarbonat mit Rückführung des anfallenden Chlorwasserstoffs und des Phenols.
**Fig. 2** zeigt ein integriertes Verfahren zur Herstellung von Diarylcarbonat (Diphenylcarbonat, DPC) und Rückführung des anfallenden Chlorwasserstoffs
**Fig. 3** zeigt ein Verfahren zur Herstellung von Diphenylcarbonat durch Direktphosgenierung von Phenol in Gegenwart von heterogenen Katalysatoren

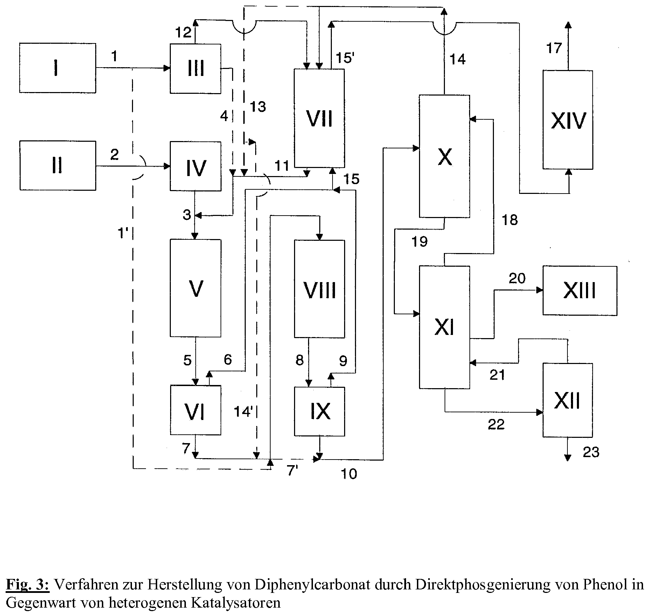

Folgende Bezugszeichen wurden in Fig. 3 verwendet:
- I: Vorratsbehälter Phenol
- II: Vorratsbehälter Phosgen
- III, IV: Wärmetauscher
- V, VIII: Reaktor
- VI; IX: Entgaser
- VII: Gegenstromapparatur
- X, XI, XII: Destillationskolonne
- XIII: Produktbehälter
- XIV: Reinigungsanlage (HCl-Absorptionsanlage)

### Beispiele

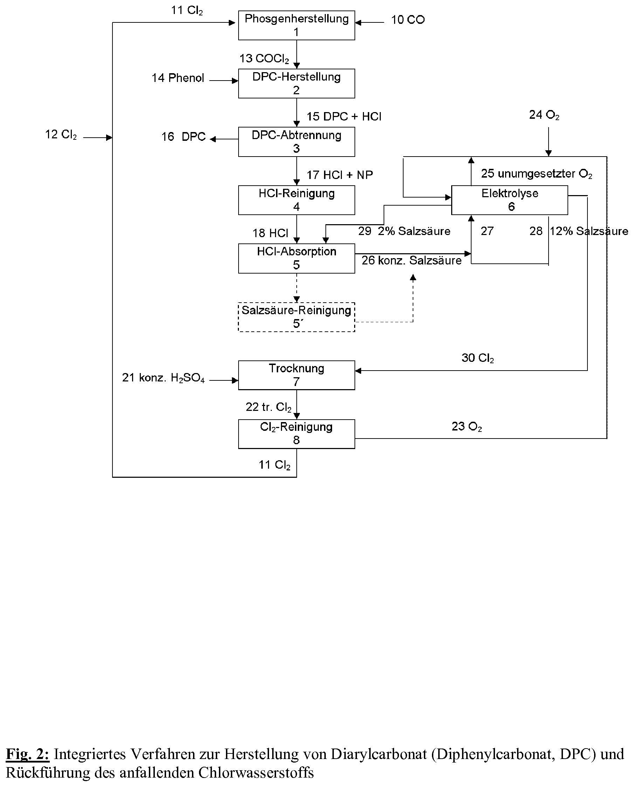

Die Beispiele sollen das erfindungsgemäße Verfahren anhand der elektrochemische Oxidation der bei der Herstellung von Diphenylcarbonat anfallenden Chlorwasserstoff zu Chlor, dessen Umsetzung zu Phosgen, das wieder in das Diphenylcarbonat-Herstellungsverfahren (DPC-Verfahren) eingesetzt werden kann (Figur 2), unter Rückführung des bei der Herstellung von Polycarbonat gebildeten Phenols in die Herstellung von Diphenylcarbonat (Figur 1), darstellen.

In einer ersten Stufe 1 der Diarylcarbonatherstellung (Figur 2) wird Chlor 11 mit Kohlenmonoxid 10 zu Phosgen 13 umgesetzt. In der folgenden Stufe 2 wird Phosgen 13 aus Stufe 1 mit einem Monophenol 14 (z.B. Phenol) zu einem Gemisch 15 aus Diarylcarbonat (z.B. Diphenylcarbonat, DPC) und Chlorwasserstoff umgesetzt, das in Stufe 3 in Diarylcarbonat 16, das verwertet wird, und HCl-Gas 17, das einer Reinigung 4 unterzogen wird, aufgetrennt wird. Das gereinigte HCl-Gas 18 wird einer HCl-Absorption 5 zugeführt.

Die konzentrierte Salzsäure 26, die dabei anfällt, wird ausgeschleust und, gegebenenfalls nach Reinigung 5' der Salzsaürelösung, einer elektrochemischen Oxidation 6 zugeführt. Die Elektrolysestufe ist eine ODC-Elektrolyse mit Gasdiffusionselektrode in der auf der Kathodenseite Sauerstoff als Reaktand und einem Rhodiumsulfid-Katalysator eingesetzt wird.

Die Konzentration der der Elektrolysezelle 6 zugeführten Salzsäure 27 beträgt 14 bis 15 Gew.-% HCl, die der aus der Elektrolysezelle 6 ablaufenden Salzsäure 28 beträgt 11 bis 13 Gew.- % HCl. Der Salzsäure-Strom 28 wird mit konzentrierter Salzsäure 26 aus der HCl-Absorptionsstufe 5 versetzt und erneut der Elektrolysezelle 6 zugeführt.

Der bei der Stufe 6 verbrauchte Sauerstoff wird durch Sauerstoff aus einer externen Quelle 24 ersetzt. Der im Kathodenraum der Elektrolysezelle nicht verbrauchte Sauerstoff 25 wird im Kreis geführt und mit frischem Sauerstoff aus einer externen Quelle 24 versetzt.

Der ebenfalls im Kathodenraum anfallende ca. 2 Gew.%-ige Salzsäurestrom 29 wird der HCl-Absorptionsstufe 5 zugeführt und dient dort als Absorptionsmittel für gasförmige Chlorwasserstoff.

Das aus der Elektrolysestufe 6 so erhaltene Gasgemisch 30 wird mit konz. Schwefelsäure 21 (96 %-ig) getrocknet (Stufe 7).

Das aus der Reinigungsstufe 8 erhaltene Chlorgas 11 kann direkt in der Phosgensynthese 1 wieder eingesetzt werden. Der bei diesem Schritt anfallende sauerstoffhaltige Strom 23 wird im Stufe 6 (Elektrolyse) eingesetzt.

Das gemäß Stufe 6 hergestellte Chlor 30 wird in einer ein- oder mehrstufigen Kühlung mittels eines Kühlaggregats, z.B. eines Röhrenwärmetauscher, abgekühlt und getrocknet.

Die Trocknung 7 kann zum Beispiel mit Hilfe eines geeigneten Trocknungsmittels in einer mit Stoffaustauschelementen bestückten Absorptionskolonne erfolgen. Ein geeignetes Trocknungsmittel kann, wie z.B. in DE 10 235 476 A beschrieben, neben Molsieben oder hygroskopischen Adsorbentien z.B. Schwefelsäure 21 sein. Die Trocknung kann ein- oder mehrstufig erfolgen. Bevorzugt erfolgt die Trocknung zweistufig, indem das zu trocknende Chlor in einer ersten Stufe mit einer Schwefelsäure geringerer Konzentration, vorzugsweise 70 bis 80 %, besonders bevorzugt 75 bis 80 %, in Kontakt gebracht wird. In einer zweiten Stufe wird die Restfeuchte mittels einer höher konzentrierten Schwefelsäure von bevorzugt 88 bis 96 %, besonders bevorzugt 92-96 %, aus dem Chlor entfernt. Das auf diese Art getrocknete Chlor 22 mit einer Restfeuchte von bevorzugt maximal 100 ppm, besonders bevorzugt maximal 20 ppm kann durch einen Tröpfchenabscheider geleitet werden, um gegebenenfalls darin noch enthaltene Schwefelsäuretröpfchen zu entfernen.

Der getrocknete Chlorgasstrom 22 wird anschließend einer Chlorreinigungstufe 8 unterzogen.

Das sehr reine Chlor 11 wird mit Chlor aus einer externe Quelle 12 ergänzt und wieder der Phosgenherstellung (Stufe 1) zugeführt.

### Beispiele 1a-h

### Herstellung von Diphenylcarbonat durch Direktphosgenierung von Phenol

### 1a) Diphenylcarbonat durch Phosgenierung von Phenol in Gegenwart von γ-Al₂O₃

Die zur Durchführung des erfindungsgemäßen Verfahrens eingesetzte Apparatur und die auftretenden Stoffströme sind schematisch in Figur 3 wiedergegeben.

Aus einem beheizten Behälter I dosiert man über Wärmetauscher III (60°C) unter Normaldruck 41,12 Gew.-Teile/h Phenol 1, frisch und/oder zurückgewonnen aus der Polycarbonatherstellung, wie in Bsp 3) beschrieben, von oben in eine auf 60°C beheizte mit Füllkörpern beschickte Gegenstromkolonne VII, in welcher eine Vermischung mit aus der Destillationskolonne X am Kopf entnommenen Phenol 14 stattfindet. Nach Durchströmen des aus den Entgasungs-apparaturen VI und IX stammenden Abgasstroms 15 durch VII wird Mischung 11, (Gewichtsverhältnis Phenol/Phosgen ca. 97/3) am Fuß ausgespeist. Aus dem Vorratsbehälter II werden 21,93 Gew.-Teile/h über Wärmetauscher IV (170°C) vorgeheiztes Phosgen 3 zusammen mit 11 in einem auf 170°C beheizten, mit 150 Vol.-Teilen γ-Aluminiumoxid gefüllten Reaktor V in Gleichstrom eingeleitet.

Das am Fuß des Reaktors austretende Produkt 5, das Phenol, Chlorameisensäurephenylester, Diphenylcarbonat und Nebenprodukte im Verhältnis 56,1/0,8/ 42,8/0,3 enthält, wird über Entgaser VI in das Abgas 6 (Gewichtsverhältnis Phenol, Phosgen, Chlorwasserstoff und Kohlendioxid 2,5/15,8/81,1/0,6) und Sumpf 7 (Gewichtsverhältnis Phenol, Chlorameisensäurephenylester, Diphenylcarbonat und Nebenprodukte 55,8/0,9/43,0/0,3) aufgetrennt.

Der im Sumpf 7 vorhandene Chlorameisensäurephenylester wird durch Nachreaktion mit vorhandenem Phenol (eventuell nach Zugabe von zusätzlichem Phenol (1' oder 14') in einem Nachreaktor VIII, ebenfalls befüllt mit γ-Aluminiumoxid (150 Vol.-Teilen) bei 180°C zu Diphenylcarbonat umgesetzt.

Das am Reaktorfuß entnommene Produkt 8 (Gewichtsverhältnis Phenol, Diphenylcarbonat und Nebenprodukte 55,4/44,3/0,3) wird über Entgaser IX in Abgas 9 (Phenol/Chlorwasserstoff) und Sumpf 10 (Gewichtsverhältnis Phenol, Diphenylcarbonat und Nebenprodukte 55,2/44,5/0,3) aufgetrennt.

Sumpf 10 wird in eine erste Destillationskolonne X eingespeist und bei ca. 80°C/16 mbar (12 mm) aufgetrennt in 57,7 Gew.-Teile/h Phenol und Sumpf 19 (Gewichtsverhältnis Phenol, Diphenylcarbonat und Nebenprodukte 0,3/99,1/0,6). Sumpf 19 wird einer zweiten Destillations-kolonne XI zugeleitet, in der über Kopf noch vorhandenes Phenol (0,14 Gew.-Teile) entfernt und in den oberen Teil der ersten Kolonne X zurückgegeben wird. Das am Fuß entnommene Produkt 22 (Gewichtsverhältnis Diphenylcarbonat/Nebenprodukte 88,6/11,4) wird in einer dritten Destillationskolonne XII bei 170°C/12mm in Kopfprodukt 21 (2,3 Gew.-Teile/h Diphenylcarbonat), das in den unteren Teil der zweiten Kolonne XI zurückgeführt wird, und Sumpf 23 (hochsiedende Nebenprodukte) getrennt. Durch seitliche Ausspeisung aus dem Gasraum der 2. Kolonne XI erhält man 46,6 Gew.-Teile/h Produkt 20 (Gewichtsverhältnis Diphenylcarbonat, Phenol 99,8/0,2), das dem Produktbehälter XIII zugeführt wird.

Abgasströme 6 und 9 werden vereinigt zu 15 (Gewichtsverhältnis Phenol, Chlorwasserstoff und Kohlendioxid 5,8/93,6/0,6) und in der Gegenstromapparatur VII durch Phenol geleitet. Das am Kopf austretende Abgas 15' wird einer Reinigungsanlage XIV zugeführt, wo das Chlorwasserstoffgas durch Ausfrieren von Phenol befreit wird..

### 1b) Phosgenierung von Phenol in Gegenwart von TiCl₄ in einer Reaktivdestillationskolonne

Man dosiert von oben in eine auf 160°C thermostatisierte Reaktivdestillationskolonne mit 10 Böden von je 18 Volumeteilen unter Normaldruck 49,9 Gew.-Teile/h aufgeschmolzenes Phenol und 0,25 Gew.-Teile/h TiCl₄ und gleichzeitig von unten 25,1 Gew.-Teile/h auf gleicher Temperatur erhitztes Phosgen in Gegenstrom.

Das am Fuß austretende Produkt, enthaltend Chlorameisensäurephenylester und Diphenylcarbonat im Gewichtsverhältnis 3 zu 97, (Phenolumsatz 69,5%, Selektivität 99,6%) wird nach Umsetzung des vorhandenen Chlorameisensäurephenylesters mit Phenol zu Diphenylcarbonat in einem Verweilzeitreaktor isoliert und durch Entgasung in Abgas und Sumpf aufgetrennt.

Der am Kopf der Reaktivdestillationsanlage austretende Abgasstrom wird mit dem Abgas aus der Entgasung vereinigt und von geringen Mengen Phosgen und Phenol durch Ausfrieren befreit.

Der Phosgenumsatz wurde gemessen durch Bestimmung des Phosgengehalts in einem im Abgasstrom eingebauten Gasmaus durch Reaktion mit Überschuß Ethanol und Korrelation des gebildeten Chlorameisensäure ethyl ester und Diethylcarbonat mit der Menge nicht abreagiertes Phosgen.

### 1c) Phosgenierung von Phenol in Gegenwart von TiCl₄ in einer Reaktivdestillationskolonne

Man dosiert von oben in eine auf 150°C thermostatisierte Reaktivdestillationsapparatur mit 20 Böden von je 43 Volumeteilen unter Normaldruck 187,3 Gew.-Teile/h aufgeschmolzenes Phenol und 0,42 Gew.-Teile/h TiCl₄ und gleichzeitig von unten 62,7 Gew.-Teile/h auf gleicher Temperatur erhitztes Phosgen in Gegenstrom.

Das am Fuß austretende Produkt, enthaltend Phenol, Chlorameisensäurephenylester, Diphenylcarbonat und Nebenprodukte (Gewichtsverhältnis 31,7/5,2/62,8/0,3) (Phosgenumsatz 83,5%, Phenolumsatz 64,8%, Selektivität 99,7%) wird nach Umsetzung des vorhandenen Chlorameisensäurephenylesters und Phenols zu Diphenylcarbonat in einem Verweilzeitreaktor isoliert und durch Entgasung in Abgas und Sumpf (Gewichtsverhältnis Phenol, Diphenylcarbonat und Nebenprodukte 29,2/70,4/0,4) aufgetrennt.

Nach Destillation erhält man 99,8%-iges Diphenylcarbonat.

Der am Kopf der Reaktivdestillationsanlage austretende Abgasstrom wird mit dem Abgas aus der Entgasung vereinigt und von geringen Mengen Phosgen und Phenol durch Ausfrieren befreit.

### 1d) Phosgenierung von Phenol in Gegenwart von AlCl₃ in einer Reaktivdestillationskolonne

Man dosiert von oben in eine auf 150°C thermostatisierte Reaktivdestillationsapparatur mit 20 Böden von je 43 Volumeteilen unter Normaldruck 399,0 Gew.-Teile/h aufgeschmolzenes Phenol und 0,61 Gew.-Teile/h AlCl₃ und gleichzeitig von unten 100,0 Gew.-Teile/h auf gleicher Temperatur erhitztes Phosgen in Gegenstrom.

Das am Fuß austretende Produkt, enthaltend Phenol, Chlorameisensäure phenylester, Diphenylcarbonat und Nebenprodukte (Gewichtsverhältnis 60,7/3,5/34,8/0,6) (Phosgenumsatz 71,6%, Phenolumsatz 35,4%, Selektivität 99%) wird nach Umsetzung des vorhandenen Chlorameisensäurephenylesters mit Phenol zu Diphenylcarbonat in einem Verweilzeitreaktor isoliert und durch Entgasung in Abgas und Sumpf (Gewichtsverhältnis Phenol, Diphenylcarbonat und Nebenprodukte 59,6/39,6/0,8) aufgetrennt.

Das Abgas wird mit dem am Kopf der Reaktivdestillationsanlage austretende Abgasstrom vereinigt und von geringen Mengen Phosgen und Phenol durch Ausfrieren befreit.

Nach Destillation bei 172°C/22 mbar wird 99,7%-iges Diphenylcarbonat erhalten.

### 1e) Phosgenierung von Phenol in Gegenwart von ZrCl₄ in einer Blasensäulekaskade

Man leitet in zwei nach einander geschaltete, auf 160°C thermostatisierte Blasensäulen von je 100 Volumeteilen befüllt mit 141,0 Gew.-Teilen aufgeschmolzenes Phenol und 0,88 Gew.-Teile/h ZrCl₄ unter Normaldruck über 3 h von unten durch eine Gasfritte 24,8 Gew.-Teile/h auf gleicher Temperatur erhitztes Phosgen ein.

Die Reaktionsmischung der 1. Blasensäule enthaltend Chlorameisensäurephenylester und Diphenylcarbonat im Gewichtsverhältnis 2 zu 98 (Phenolumsatz 83,4%, Selektivität 98,3%) wird nach Umsetzung des vorhandenen Chlorameisensäure-phenylesters mit Phenol zu Diphenylcarbonat in einem Verweilzeitreaktor isoliert und durch Entgasung in Abgas und Sumpf aufgetrennt, der durch Destillation aufgetrennt wird.

Der am Kopf der 1. Blasensäule austretende Abgasstrom wird durch eine Gasfritte in die 2. Blasensäule geleitet.enthält In der 2. Blasensäule ist nach 3h der Phenolumsatz 22,0%.

### 1f) Phosgenierung von Phenol in Gegenwart von TiCl₄ in einer Blasensäulekaskade

Nach Durchführung der Direktphosgenierung wie beschrieben in 1e) aber mit 0,71 Gew.-Teilen TiCl₄ enthält die Reaktionsmischung in der 1. Blasensäule Chlorameisensäurephenylester und Diphenylcarbonat im Gewichtsverhältnis 0,6 zu 99,4 (Phenolumsatz 80,2%, Selektivität 99,5%). In der 2. Blasensäule ist der Phenolumsatz 12,3%.

### 1g) Phosgenierung von Phenol in Gegenwart von Ti(OC₆H₅)₄ in einer Blasensäulekaskade

Nach Durchführung der Direktphosgenierung wie beschrieben in 1e) aber mit 1,57 Gew.-Teilen Ti(OC₆H₅)₄ enthält die Reaktionsmischung in der 1. Blasensäule Chlorameisensäurephenylester und Diphenylcarbonat im Gewichtsverhältnis 0,7 zu 99,3 (Phenolumsatz 81,5%, Selektivität 99,4%). In der 2. Blasensäule ist der Phenolumsatz 17,4%.

### 1h) Phosgenierung von Phenol in Gegenwart von TiCl₄ in der Gasphase

Man dosiert von oben in eine auf 235°C thermostatisierte Rohrreaktorsapparatur mit Glaswolle unter Normaldruck 25,0 Gew.-Teile/h aufgeschmolzenes Phenol, 0,61 Gew.-Teile/h TiCl₄ und gleichzeitig 12,5 Gew.-Teile/h auf gleicher Temperatur erhitztes Phosgen.

Das am Fuß austretende Produkt, enthaltend Chlorameisensäurephenylester und Diphenylcarbonat im Gewichtsverhältnis 0,6 zu 99,4 (Phosgenumsatz 87%, Phenolumsatz 59,8%, Selektivität 99,9%) wird nach Umsetzung des vorhandenen Chlorameisen-säurephenylesters mit Phenol zu Diphenylcarbonat in einem Verweilzeitreaktor isoliert und durch Entgasung in Abgas und Sumpf aufgetrennt.

Der bei der kontinuierlichen Herstellung von Diarylcarbonaten durch Reaktion von Monophenolen und Phosgen in Gegenwart von Katalysatoren anfallende Chlorwasserstoff kann nach Entfernung der geringfügigen Phosgen- und Monophenolverunreinigungen zB. durch Ausfrieren ohne aufwendige Reinigung gegebenenfalls nach Behandlung mit Aktivkohle einer elektrochemischen Oxidation zugeführt werden.

### Beispiel 2

### Rückführung von Chlorwasserstoff aus der Diphenylcarbonatherstellung durch elektrochemische Oxidation mit Sauerstoff

### a) Elektrochemische Oxidation des Chlorwasserstoffs mit einer Gasdiffusionselektrode als Sauerstoffverzehrkathode

Ein Strom von 8,16 Gew.-Teile/h gereinigten Chlorwasserstoff aus der Diphenylcarbonatherstellung 1 wird einer HCl-Absorption zugeführt. Hierzu wird in die Salzsäure 26 aus der HCl-Absorption ein Teilstrom des aus der Elektrolyse kommenden an Salzsäure abgereichterten Anolytsäure-Stromes 28 eingeleitet. Es werden 32,1 Gew.-Teile/h der an Salzsäure abgereicherten Anolyt-Säure 28 mit einer HCl-Konzentration von 12,2 Gew.-% der HCl-Absorption zugeführt. In dieser HCl-Absorption-Einheit wird eine 30 Gew.%-ige Salzsäure 26 hergestellt, die mit dem Rest der abgereicherten Anolytsäure 28 vereinigt und erneut der Elektrolysezelle zugeführt wird. 2,96 Gew.-Teile/h der abgereicherten Anolytsäure 28 werden aus dem Anolytsäure-Kreislauf ausgeschleust. (nicht in der Figur gezeichnet)

Die Elektrolyse wird mit einer Stromdichte von 5 kA/m² bei 55°C und einer Spannung von 1,39 V betrieben. Als Anoden- und Kathodenwerkstoff wird ein Palladium-stabilisiertes Titan eingesetzt. An der Rutheniumoxid-beschichteten Anode der Fa. DENORA, Deutschland, wird 10,1 Gew.-Teile/h Chlor entwickelt. Anoden- und Kathoden-Halbschale werden durch eine Ionenaustauschermembran der Fa. DUPONT, Typ Nafion 324 getrennt. Als Kathode wird eine Sauerstoffverzehrkathode der Fa. ETEK eingesetzt, die einen Rhodiumsulfid-geträgerten Katalysator enthält. Sauerstoff wird dem Kathoden-Halbelement mit 100 % Überschuss zugeführt, d.h. mit 9,17 Gew.-Teile/h. Der Sauerstoff wird in die Elektrolyse recycliert, ein Purge-Strom von 1 % der Feedmenge wird hinter der Elektrolyse abgeführt verwendet. Der Druck in der Anodenhalbzelle ist höher als der in der Kathodenhalbzelle. Der Differenzdruck beträgt 200 mbar. Der Kathodenhalbzelle wird ein Kondensatstrom von 8,8 Gew.-Teile/h entnommen.

Die Elektrolyseeinheit besteht aus 615 Elektrolyseelementen, wobei ein Element aus einer Anodenhalbschale mit Anode, einer Ionenaustauschermembran und einer Kathodenhalbschale mit Sauerstoffverzehrkathode besteht.

### Beispiel 3

### Herstellung von Polycarbonat

Aus einer Vorlage werden 8.600 Gew.-Teile/h Schmelzegemisch, bestehend aus 4.425 Gew.-Teilen/h Diphenylcarbonat, hergestellt wie beschrieben in 1a) oder 1b), und 4.175 Gew.-Teilen/h Bisphenol A, unter Zusetzen von 0,52 Gew.-Teilen/h des Phenoladdukts von Tetraphenylphosphoniumphenolat mit 65,5 % Tetraphenylphosphoniumphenolat/h gelöst in 4,5 Gew.-Teile/h Phenol/h durch einen Wärme-tauscher gepumpt, auf 190°C erwärmt und durch eine Verweilkolonne bei 12 bar und 190°C geführt. Die mittlere Verweilzeit beträgt 50 Minuten.

Die Schmelze wird dann über ein Entspannungsventil in einen unter 200 mbar stehenden Abscheider geleitet. Die abfließende Schmelze wird in einem, ebenfalls unter 200 mbar stehenden, Fallfilmver-dampfer wieder auf 189°C erwärmt und in einer Vorlage aufgefangen. Nach einer Verweilzeit von 20 Minuten wird die Schmelze in die nächsten drei, gleichartig aufgebauten Stufen gepumpt. Die Bedingungen in der 2. / 3. / 4. Stufe sind 100 / 74 / 40 mbar, 218 / 251 / 276°C und 20 / 10 / 10 Minuten. Das entstandene Oligomere hat eine relative Viskosität von 1,09. Alle Brüden werden über Druckregelungen in eine unter Vakuum stehende Kolonne geführt und als Kondensate abgeleitet.

Danach wird das Oligomer in einem sich anschliessenden Korbreaktor bei 278°C und 3,0 mbar bei einer Verweilzeit von 45 Minuten zu einem höhermolekularen Produkt aufkondensiert. Die relative Viskosität beträgt 1,195. Die Brüden werden kondensiert.

Vom Schmelzestrom, der in einen weiteren Korbreaktor geleitet wird, wird mittels einer Zahnradpumpe ein Teilstrom von 150 Gew.-Teilen/h Schmelze abgezweigt, mit 0,185 Gew.-Teilen/h einer 5 %-igen wässrigen Phosphorsäure versetzt, über einen statischen Mischer mit einem Länge-zu-Durchmesser-Verhältnis von 20 gerührt und wieder in den Hautschmelzestrom zurückgeleitet. Direkt nach dem Zusammentreffen wird die Phosphorsäure im gesamten Schmelzestrom mittels eines weiteren statischen Mischers homogen verteilt.

Die so behandelte Schmelze wird in einem weiteren Korbreaktor bei 284°C, 0,7 mbar und bei einer mittleren Verweilzeit von 130 Minuten weiter den Prozessbedingungen ausgesetzt, ausgetragen und granuliert.

Die Brüden werden in der Vakuumanlage und dahinter kondensiert.

Das erhaltene Polycarbonat hat folgende Kennzahlen: relative Viskosität 1,201 / phenolische OH 255 [ppm] / DPC 71 [ppm] / BPA 6 [ppm] / Phenol 56 [ppm].

Das abdestillierte Phenol kann wieder in die Diphenylcarbonatherstellung gemäß Schritt (b), wie beschrieben in den Beispielen 1a-h), rückgeführt werden.

## Patentansprüche

1. Verfahren zur Herstellung von Diarylcarbonat umfassend folgende Schritte:
(a) Herstellung von Phosgen durch Umsetzung von Chlor mit Kohlenmonoxid,
(b) Umsetzung des gemäß Schritt (a) gebildeten Phosgens mit wenigstens einem Monophenol in Gegenwart eines Katalysators, und gegebenenfalls organischem Lösungsmittel unter Bildung wenigstens eines Diarylcarbonats und von Chlorwasserstoff,
(c) Abtrennung und Aufarbeitung des in Schritt (b) gebildeten Diarylcarbonats
(d) Abtrennung und gegebenenfalls Reinigung des gemäß Schritt (b) gebildeten Chlorwasserstoffs
(e) Herstellung einer wässrigen Lösung des Chlorwasserstoffs
(f) gegebenenfalls Reinigung der wässrigen Lösung des Chlorwasserstoffs,
(g) elektrochemische Oxidation wenigstens eines Teils der wässrigen Chlorwasserstoff-Lösung aus (e) oder (f) zu Chlor unter Bildung von Wasser
(h) Rückführung wenigstens eines Teils des gemäß Schritt (g) hergestellten Chlors in die Herstellung von Phosgen gemäß Schritt (a),
wobei die elektrochemische Oxidation in Schritt (g) unter Verwendung einer Gasdiffusionselektrode als Sauerstoffverzehrkathode erfolgt.

2. Verfahren nach Anspruch 1, **dadurch gekennzeichnet, dass** eine Reinigung der wässrigen Lösung des Chlorwasserstoffs aus Schritt (e) stattfindet, bevor die Lösung der elektrochemischen Oxidation (g) unterworfen wird.

3. Verfahren gemäß Anspruch 1 oder 2, **dadurch gekennzeichnet, dass** die Abtrennung gemäß Schritt (d) des gemäß Schritt (c) gebildeten Chlorwasserstoffs von Phosgen mittels Verflüssigung umfasst.

4. Verfahren gemäß einem der Ansprüche 1 bis 3, **dadurch gekennzeichnet, dass** gemäß Schritt (d) eine Reinigung des Chlorwasserstoffs mittels Ausfrieren erfolgt.

5. Verfahren gemäß einem der Ansprüche 1 bis 4, **dadurch gekennzeichnet, dass** die Herstellung der wässrigen Chlorwasserstofflösung gemäß Schritt (e) durch Absorption in einer wässrigen Lösung von Chlorwasserstoff, bevorzugt mit einer Konzentration von 15 bis 20 Gew.%, erfolgt.

6. Verfahren gemäß einem der Ansprüche 1 bis 5, **dadurch gekennzeichnet, dass** gemäß Schritt (f) eine Strippung der wässrigen Chlorwasserstofflösung mit Dampf und/oder eine Behandlung mit Aktivkohle erfolgt.

7. Verfahren gemäß einem der Ansprüche 1 bis 6, **dadurch gekennzeichnet, dass** gemäß Schritt (f) zusätzlich eine Entfernung von Eisen-, Silizium- und / oder Aluminium-Verbindungen aus der wässrigen Chlorwasserstofflösung mittels Ionenaustauscher erfolgt.

8. Verfahren gemäß einem der Ansprüche 1 bis 7, **dadurch gekennzeichnet, dass** die elektrochemische Oxidation der wässrigen Chlorwasserstoff-Lösung gemäß Schritt (g) in einer Elektrolysezelle erfolgt, in welcher der Anodenraum und der Kathodenraum durch eine Ionenaustauschermembran getrennt sind.

9. Verfahren gemäß einem der Ansprüche 1 bis 8, **dadurch gekennzeichnet, dass** die elektrochemische Oxidation der wässrigen Chlorwasserstoff-Lösung gemäß Schritt (g) in einer Elektrolysezelle erfolgt, in welcher der Anodenraum und der Kathodenraum durch ein Diaphragma getrennt sind.

10. Verfahren gemäß einem der Ansprüche 1 bis 9, **dadurch gekennzeichnet, dass** die Kathode Rhodiumsulfid enthält.

11. Verfahren gemäß einem der Ansprüche 1 bis 10, **dadurch gekennzeichnet, dass** der wässrigen Chlorwasserstoff-Lösung vor der elektrochemischen Oxidation gemäß Schritt (g) Metallionen aus der Gruppe der Platinmetalle, zugesetzt werden.

12. Verfahren gemäß einem der Ansprüche 1 bis 11, **dadurch gekennzeichnet, dass**, Chlor gemäß Schritt (a) zu Phosgen umgesetzt und das so erhaltene Phosgen zur Umsetzung mit Phenol zu Diphenylcarbonat gemäß Schritt (b) eingesetzt wird.

13. Verfahren gemäß einem der Ansprüche 1 bis 12, **dadurch gekennzeichnet, dass** das Diarylcarbonat mit einem Bisphenol zum Oligo-/Polycarbonat und einem Monophenol umgesetzt und das so erhaltene Monophenol zur Umsetzung mit Phosgen zu Diphenylcarbonat gemäß Schritt (b) eingesetzt wird.

14. Verfahren gemäß einem der Ansprüche 1 bis 13, **dadurch gekennzeichnet, dass** es sich beim hergestellten Diarylcarbonat um Diphenylcarbonat handelt.

## Claims

1. Process for preparing diaryl carbonate, which comprises the following steps:
(a) preparation of phosgene by reaction of chlorine with carbon monoxide,
(b) reaction of the phosgene formed in step (a) with at least one monophenol in the presence of a catalyst and optionally organic solvent to form at least one diaryl carbonate and hydrogen chloride,
(c) isolation and work-up of the diaryl carbonate formed in step (b),
(d) isolation and optionally purification of the hydrogen chloride formed in step (b),
(e) preparation of an aqueous solution of the hydrogen chloride,
(f) optionally purification of the aqueous solution of the hydrogen chloride,
(g) electrochemical oxidation of at least part of the aqueous hydrogen chloride solution from (e) or (f) to chlorine with formation of water,
(h) recirculation of at least part of the chlorine prepared in step (g) to the preparation of phosgene in step (a),
where the electrochemical oxidation in step (g) is carried out using a gas diffusion electrode as oxygen-consuming cathode.

2. Process according to Claim 1, **characterized in that** a purification of the aqueous solution of the hydrogen chloride from step (e) takes place before the solution is subjected to the electrochemical oxidation (g).

3. Process according to Claim 1 or 2, **characterized in that** the removal as per step (d) of the hydrogen chloride formed as per step (c) comprises phosgene by means of liquefaction.

4. Process according to any of Claims 1 to 3, **characterized in that** a purification of the hydrogen chloride by means of freezing-out is carried out in step (d).

5. Process according to any of Claims 1 to 4, **characterized in that** the preparation of the aqueous hydrogen chloride solution in step (e) is carried out by absorption in an aqueous solution of hydrogen chloride, preferably a solution having a concentration of from 15 to 20% by weight.

6. Process according to any of Claims 1 to 5, **characterized in that** stripping of the aqueous hydrogen chloride solution with stream and/or treatment with activated carbon is carried out in step (f).

7. Process according to any of Claims 1 to 6, **characterized in that** removal of iron compounds, silicon compounds and/or aluminium compounds from the aqueous hydrogen chloride solution by means of ion exchangers is additionally carried out in step (f).

8. Process according to any of Claims 1 to 7, **characterized in that** the electrochemical oxidation of the aqueous hydrogen chloride solution in step (g) is carried out in an electrolysis cell in which the anode space and the cathode space are separated by an ion-exchange membrane.

9. Process according to any of Claims 1 to 8, **characterized in that** the electrochemical oxidation of the aqueous hydrogen chloride solution in step (g) is carried out in an electrolysis cell in which the anode space and the cathode space are separated by a diaphragm.

10. Process according to any of Claims 1 to 9, **characterized in that** the cathode contains rhodium sulphide.

11. Process according to any of Claims 1 to 10, **characterized in that** metal ions from the group of the platinum metals are added to the aqueous hydrogen chloride solution before the electrochemical oxidation in step (g).

12. Process according to any of Claims 1 to 11, **characterized in that** chlorine is converted into phosgene as per step (a) and the resulting phosgene is used for the reaction with phenol to form diphenyl carbonate in step (b).

13. Process according to any of Claims 1 to 12, **characterized in that** the diaryl carbonate is reacted with a bisphenol to form the oligocarbonate/polycarbonate and a monophenol and the resulting monophenol is used for the reaction with phosgene to form diphenyl carbonate in step (b).

14. Process according to any of Claims 1 to 13, **characterized in that** the diaryl carbonate prepared is diphenyl carbonate.

## Revendications

1. Procédé de fabrication de carbonate de diaryle comprenant les étapes suivantes :
(a) la fabrication de phosgène par mise en réaction de chlore avec du monoxyde de carbone,
(b) la mise en réaction du phosgène formé selon l'étape (a) avec au moins un monophénol en présence d'un catalyseur, et éventuellement d'un solvant organique, avec formation d'au moins un carbonate de diaryle et de chlorure d'hydrogène,
(c) la séparation et le traitement du carbonate de diaryle formé à l'étape (b),
(d) la séparation et éventuellement la purification du chlorure d'hydrogène formé selon l'étape (b),
(e) la fabrication d'une solution aqueuse de chlorure d'hydrogène,
(f) éventuellement la purification de la solution aqueuse de chlorure d'hydrogène,
(g) l'oxydation électrochimique d'au moins une partie de la solution aqueuse de chlorure d'hydrogène de (e) ou (f) en chlore avec formation d'eau,
(h) le recyclage d'au moins une partie du chlore fabriqué selon l'étape (g) dans la fabrication de phosgène selon l'étape (a),
l'oxydation électrochimique à l'étape (g) ayant lieu en utilisant une électrode à diffusion de gaz en tant que cathode de consommation d'oxygène.

2. Procédé selon la revendication 1, **caractérisé en ce qu'**une purification de la solution aqueuse du chlorure d'hydrogène de l'étape (e) a lieu avant que la solution ne soit soumise à l'oxydation électrochimique (g).

3. Procédé selon la revendication 1 ou 2, **caractérisé en ce que** la séparation selon l'étape (d) du chlorure d'hydrogène formé selon l'étape (c) du phosgène comprend une liquéfaction.

4. Procédé selon l'une quelconque des revendications 1 à 3, **caractérisé en ce qu'**une purification du chlorure d'hydrogène par congélation a lieu selon l'étape (d).

5. Procédé selon l'une quelconque des revendications 1 à 4, **caractérisé en ce que** la fabrication de la solution aqueuse de chlorure d'hydrogène selon l'étape (e) a lieu par absorption dans une solution aqueuse de chlorure d'hydrogène, de préférence en une concentration de 15 à 20 % en poids.

6. Procédé selon l'une quelconque des revendications 1 à 5, **caractérisé en ce qu'**une extraction de la solution aqueuse de chlorure d'hydrogène avec de la vapeur et/ou un traitement avec du charbon actif ont lieu selon l'étape (f).

7. Procédé selon l'une quelconque des revendications 1 à 6, **caractérisé en ce qu'**une élimination de composés de fer, de silicium et/ou d'aluminium de la solution aqueuse de chlorure d'hydrogène au moyen d'échangeurs d'ions a en outre lieu selon l'étape (f).

8. Procédé selon l'une quelconque des revendications 1 à 7, **caractérisé en ce que** l'oxydation électrochimique de la solution aqueuse de chlorure d'hydrogène selon l'étape (g) a lieu dans une cellule d'électrolyse dans laquelle la chambre d'anode et la chambre de cathode sont séparées par une membrane échangeuse d'ions.

9. Procédé selon l'une quelconque des revendications 1 à 8, **caractérisé en ce que** l'oxydation électrochimique de la solution aqueuse de chlorure d'hydrogène selon l'étape (g) a lieu dans une cellule d'électrolyse dans laquelle la chambre d'anode et la chambre de cathode sont séparées par un diaphragme.

10. Procédé selon l'une quelconque des revendications 1 à 9, **caractérisé en ce que** la cathode contient du sulfure de rhodium.

11. Procédé selon l'une quelconque des revendications 1 à 10, **caractérisé en ce que** des ions métalliques des métaux du groupe platine sont ajoutés à la solution aqueuse de chlorure d'hydrogène avant l'oxydation électrochimique selon l'étape (g).

12. Procédé selon l'une quelconque des revendications 1 à 11, **caractérisé en ce que** du chlore est transformé en phosgène selon l'étape (a) et le phosgène ainsi obtenu est utilisé pour la réaction avec le phénol pour former du carbonate de diphényle selon l'étape (b).

13. Procédé selon l'une quelconque des revendications 1 à 12, **caractérisé en ce que** le carbonate de diaryle est mis en réaction avec un bisphénol pour former l'oligo-/polycarbonate et un monophénol, et le monophénol ainsi obtenu est utilisé pour la réaction avec le phosgène pour former du carbonate de diphényle selon l'étape (b).

14. Procédé selon l'une quelconque des revendications 1 à 13, **caractérisé en ce que** le carbonate de diaryle fabriqué est le carbonate de diphényle.
